# FASCICULE DE BREVET EUROPEEN

(11) **EP 1 088 546 B1**
(45) Date de publication et mention de la délivrance du brevet: **14.07.2004**
(21) Numéro de dépôt: 00402659.7
(22) Date de dépôt: 26.09.2000
(51) Int. Cl.: A61K 7/075, A61K 7/06

(54) **Composition de traitement antipelliculaire des cheveux et du cuir chevelu, à base d'un agent antipelliculaire et d'un terpolymère acrylique**
Zusammensetzung zur Behandlung der Haare und der Kopfhaut gegen Schuppen, die ein Antischuppenmittel und ein Acrylterpolymer enthält
Composition for treating dandruff in hair and scalp based on an antidandruff agent and an acrylic terpolymer

(30) Priorité: 29.09.1999 FR 9912165
(43) Date de publication de la demande: 04.04.2001
(73) Titulaire: L'OREAL, 75008 Paris (FR)
(72) Inventeur: Maurin, Véronique, 75016 Paris (FR); Beauquey, Bernard, 92110 Clichy (FR)
(74) Mandataire: Casalonga, Axel

(56) Documents cités:
- EP-A- 0 824 914
- EP-A- 0 825 200
- FR-A- 2 774 901

## Description

La présente invention concerne d'une manière générale des compositions de traitement antipelliculaire des cheveux et du cuir chevelu, à base d'un agent antipelliculaire et d'un terpolymère acrylique, ainsi qu'un procédé de traitement mettant en oeuvre ces compositions.

Les désordres desquamatifs du cuir chevelu tels que les pellicules sont liés à la présence d'une levure caractéristique appelée le Malassezia ovalis, levure anciennement dénommée Pityrosporum (P. ovale et P. orbiculare). Le traitement usuel des pellicules fait intervenir l'utilisation d'agents antipelliculaires, notamment des agents antifongiques et/ou antibactériens dans un milieu, tel qu'un shampooing, un gel ou une lotion, qui est apte à répartir ces agents et à les déposer sur les téguments.

Il a été constaté que les traitements utilisant ces agents antipelliculaires présentaient souvent l'inconvénient d'altérer les fibres kératiniques, et plus particulièrement les fibres kératiniques sensibilisées, en diminuant leurs performances cosmétiques, notamment en les rendant plus rêches et plus chargées.

Il existe donc un besoin d'une composition cosmétique antipelliculaire comme par exemple un shampooing, une lotion ou un gel, qui permette d'obtenir des performances cosmétiques acceptables, notamment au niveau de la douceur des cheveux.

La présente invention a pour objet des compositions de traitement antipelliculaire des cheveux et du cuir chevelu, conférant aux cheveux les mêmes propriétés cosmétiques que celles obtenues lors de l'utilisation d'une composition de traitement des matières kératiniques ne comprenant pas d'agent antipelliculaire.

La demanderesse a découvert, de façon surprenante, qu'il était possible de formuler des compositions de traitement des matières kératiniques, notamment des lotions et shampooings, ayant les propriétés recherchées, en utilisant dans ces compositions un agent antipelliculaire associé à un terpolymère acrylique spécifique, défini ci-après. En effet, il a été constaté que l'utilisation des compositions de la présente invention permettait d'améliorer les propriétés cosmétiques des cheveux et du cuir chevelu, particulièrement en apportant plus de douceur et de souplesse aux cheveux sensibilisés mouillés et en apportant plus de douceur, de lissage aux cheveux séchés. L'utilisation des compositions de l'invention permet aussi d'obtenir une chevelure présentant au regard un aspect plus lisse.

Il a été également constaté que les associations de l'invention présentaient une bonne tolérance cutanée et facilitaient le démêlage des cheveux séchés.

D'autres objets de l'invention apparaîtront à la lumière de la description et des exemples qui suivent.

Selon l'invention, les compositions de traitement antipelliculaire des cheveux et du cuir chevelu sont essentiellement caractérisées en ce qu'elles comprennent dans un milieu cosmétiquement acceptable au moins un agent antipelliculaire spécifique défini ci-après et au moins un terpolymère acrylique constitué:
- de 5 à 80% en poids, préférentiellement de 15 à 70% en poids et plus préférentiellement de 40 à 70% en poids, d'un monomère acrylate (a) choisi parmi un acrylate d'alkyle en C₁-C₆ et un méthacrylate d'alkyle en C₁-C₆;
- de 5 à 80% en poids, préférentiellement de 10 à 70% en poids et plus préférentiellement de 20 à 60% en poids, d'un monomère (b) choisi parmi un composé vinylique héterocyclique contenant au moins un atome d'azote ou de soufre, un (méth)acrylamide, un (méth)acrylate de mono- ou di-(C₁-C₄)alkylamino(C₁-C₄)alkyle et un mono ou di-(C₁-C₄)alkylamino (C₁-C₄) alkyl (méth)acrylamide;
- de 0,1 à 30% en poids, préférentiellement de 0,1 à 10% en poids d'un monomère (c) choisi parmi :
   un uréthane produit par réaction entre un isocyanate insaturé monoéthylénique et un agent tensioactif non ionique englobant un copolymère séquencé d'oxyde de 1,2-butylène et d'oxyde d'éthylène à extrémité alcoxy en C₁₋₄ ;
   un monomère tensioactif insaturé éthylénique copolymérisable obtenu par condensation d'un tensioactif non-ionique avec un acide carboxylique insaturé α,β-éthylénique ou son anhydride;
   un monomère tensioactif choisi parmi les produits de réaction de type urée d'un monoisocyanate insaturé monoéthylénique avec un tensioactif non-ionique présentant une fonction amine;
   un éther de (méth)allyle de formule CH₂=CR₁CH₂OAₘBₙAₚR₂ dans lequel R₁ désigne un atome d'hydrogène ou un groupe méthyle, A désigne un groupement propylèneoxy ou butylèneoxy, B désigne éthylèneoxy, n est égal à zéro ou désigne un nombre entier inférieur ou égal à 200 et préférentiellement inférieur ou égal à 100, m et p désignent zéro ou un nombre entier inférieur à n et R₂ est un groupe hydrophobe d'au moins 8 atomes de carbone et préférentiellement en C₈-C₃₀; et
   un monomère non-ionique de type uréthane produit par réaction d'un tensioactif non ionique monohydrique avec un isocyanate insaturé monoéthylénique;
   les pourcentages en poids de monomères étant basés sur le poids total des monomères constituant le terpolymère.

Selon un mode de réalisation de l'invention, le terpolymère acrylique est présent à raison de 0,01 à 20 % en poids de matière active (M.A.), de préférence 0,1 à 10 % en poids par rapport au poids total de la composition.

Des monomères acrylates (a) préférés comprennent notamment les acrylates d'alkyle en C₂-C₆. L'acrylate d'éthyle est tout particulièrement préféré.

Comme exemples de monomères (b) préférés, il faut citer le méthacrylate de N,N-diméthylaminoéthyle (DMAEMA), l'acrylate de N,N-diéthylaminoéthyle, le méthacrylate de N,N-diéthylaminoéthyle, l'acrylate de N-t-butylaminoéthyle, le méthacrylate de N-t-butylaminoéthyle, le N,N-diméthylaminopropyl-acrylamide, le N,N-diméthylaminopropyle-méthacrylamide, le N,N-diéthylaminopropyl-acrylamide et le N,N-diéthylaminopropyl-méthacrylamide. Le méthacrylate de N,N-diméthylaminoéthyle est tout particulièrement préféré.

Les monomères (c) préférés sont les monomères tensio-actifs insaturés éthyléniques copolymérisables obtenus par condensation d'un tensioactif non-ionique avec un acide carboxylique insaturé α,β-éthylénique ou son anhydride, de préférence les acides mono ou dicarboxyliques en C₃-C₄ ou leurs anhydrides et plus particulièrement l'acide acrylique, l'acide méthacrylique, l'acide crotonique, l'acide maléique, l'anhydride maléique et tout particulièrement l'acide itaconique et l'anhydride itaconique.

Les monomères (c) particulièrement préférés correspondent aux monomères tensioactifs insaturés éthyléniques copolymérisables obtenus par condensation d'un tensioactif non-ionique avec l'acide itaconique. Parmi les tensioactifs non-ioniques, on peut citer notamment les alcools gras en C₁₀-C₃₀ alkoxylés avec 2 à 100, et de préférence de 5 à 50 moles d'oxyde d'alkylène, comme par exemple les éthers de polyéthylène glycol et d'alcools gras en C₁₀-C₃₀ et plus particulièrement les éthers de polyéthylène glycol et d'alcool cétylique, dénommés CETETH dans le dictionnaire CTFA, 7ème édition, 1997.

Des méthodes conventionnelles pour préparer ces terpolymères acryliques sont connues de l'homme du métier. De telles méthodes incluent la polymérisation en solution, la polymérisation par précipitation et la polymérisation en émulsion par exemple. Des terpolymères conformes à l'invention et leurs méthodes de préparation sont notamment décrits dans les demandes EP-A-0824914 et EP-A-0825200.

Parmi ces terpolymères, on préfère utiliser en particulier le polymère "STRUCTURE ® PLUS" vendu par la Société NATIONAL STARCH, qui est constitué d'acrylates, d'amino(méth)acrylates et d'itaconate d'alkyle en C₁₀-C₃₀ polyoxyéthyléné à 20 moles d'oxyde d'éthylène sous forme de dispersion aqueuse à 20% de M.A..

En plus de ces monomères, le terpolymère peut contenir d'autres monomères qui permettent de réticuler ledit terpolymère. Ces monomères sont utilisés dans des proportions assez faibles, jusqu'à 2% en poids par rapport au poids total des monomères utilisés pour préparer le terpolymère. De tels monomères de réticulation comprennent des monomères aromatiques portant plusieurs substituants vinyle, des monomères alicycliques portant plusieurs substituants vinyle, des esters bi-fonctionnels d'acide phtalique, des esters bi-fonctionnels d'acide méthacrylique, des esters multifonctionnels d'acide acrylique, le N-méthylène-bis-acrylamide et des monomères aliphatiques portant plusieurs substituants vinyle tels que des diènes, triènes et tétraènes. Des monomères de réticulation peuvent notamment être des divinyl-benzènes, des trivinyl-benzènes, le 1,2,4-trivinylcyclohexène, le 1,5-hexadiène, le 1,5,9-décatriène, des 1,9-décadiènes, des 1,5-heptadiènes, des di-allyl phthalates, de l'éthylène glycol diméthacrylate, des polyéthylène glycol diméthacrylates, des penta- et tétra-acrylates, des triallyl pentaérythritols, des octaallyl saccharoses, des cycloparaffines, des cyclooléfines et du N-méthylène-bis-acrylamide.

L'agent antipelliculaire des compositions de la présente invention est choisi parmi :
1) le chlorure de benzéthonium, le chlorure de benzalkonium, la chlorhexidine, la chloramine T, la chloramine B, la 1,3-dibromo 5,5-diméthylhydantoïne, la 1,3-dichloro 5,5-diméthylhydantoïne, la 3-bromo 1-chloro 5,5-diméthylhydantoïne, le N-chlorosuccinimide;
2) les dérivés de 1-hydroxy-2-pyridone représentés par la formule (I): 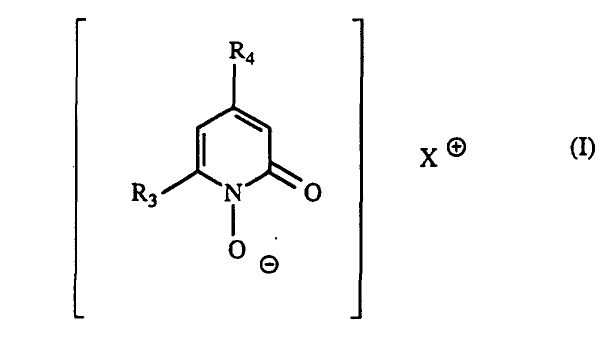 dans laquelle R₃ représente un groupe alkyle ayant de 1 à 17 atomes de carbone, alkényle ayant de 2 à 17 atomes de carbone, un groupe cycloalkyle ayant de 5 à 8 atomes de carbone, un groupe bicycloalkyle ayant de 7 à 9 atomes de carbone; un groupe cycloalkyl (-alkyle), un groupe aryle, un groupe aralkyle avec un alkyle ayant de 1 à 4 atomes de carbone, un groupe arylalkényl avec un alkényle ayant de 2 à 4 atomes de carbone, aryloxyalkyle ou arylmercaptoalkyle avec un alkyle ayant de 1 à 4 atomes de carbone, un groupe furylalkényl avec un alkényle ou un furyle ayant de 2 à 4 atomes de carbone, un groupe alkoxy ayant de 1 à 4 atomes de carbone, un groupe nitro, un groupe cyano ou un atome d'halogène.
   R₄ représente un atome d'hydrogène, un groupe alkyle en C₁-C₄, un groupe alkényle en C₂-C₄, un atome d'halogène, un groupe phényle, un groupe benzyle; X représente une base organique, un ion de métal alcalin ou alcalinoterreux, un ion ammonium.
   Des composés de formules (I) sont par exemple le 1-hydroxy-4-méthyl-2-pyridone, le 1-hydroxy-6-méthyl-2-pyridone, le 1-hydroxy-4,6-diméthyl-2-pyridone, le 1-hydroxy-4-méthyl-6-(2,4,4-triméthylpentyl)-2-pyridone, le 1-hydroxy-4-méthyl-6-cyclohexyl-2-pyridone, le 1-hydroxy-4-méthyl-6-(méthyl-cyclohexyl)-2-pyridone, le 1-hydroxy-4-méthyl-6-2-bicyclo[2,2,1]heptyl)-2-pyridone, le 1-hydroxy-4-methyl-4-(4-méthyl-phényl)-2-pyridone, le 1-hydroxy-4-methyl-6-[1-(4-nitrophenoxy)-butyl]-2-pyridone, le 1-hydroxy-4-méthyl-6-(4-cyanophenoxymethyl)-2-pyridone, le 1-hydroxy-4-methyl-6-(phenylsulfonylmethyl)-2-pyridone et le 1-hydroxy-4-méthyl-6-(4-bromo-benzyl)-2-pyridone.
   Un composé de formule (I) particulièrement préféré est celui pour lequel R₃ désigne le radical 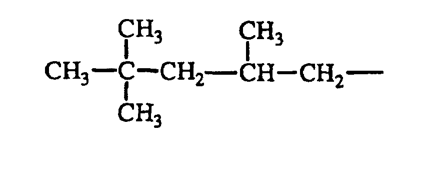 et R₄ désigne méthyle
   et X⁺ désigne N⁺H₃CH₂CH₂OH
   Ce composé est par exemple commercialisé sous la dénomination Octopirox (1-hydroxy-4-méthyl-6-(2,4,4-triméthylpentyl)-2-pyridone, sel de monoéthanolamine) par la société HOECHST.
3) les trihalogéno carbamides de formule (II) suivante: 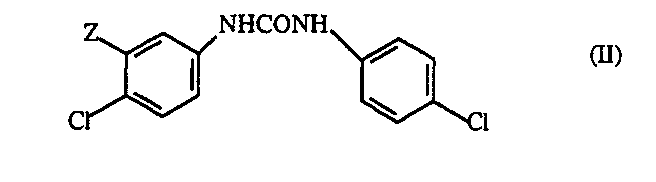 dans laquelle Z représente un atome d'halogène comme le chlore ou un groupement trihalogénoalkyle en C₁-C₄ tel que CF3;
4) le triclosan représenté par la formule (III) 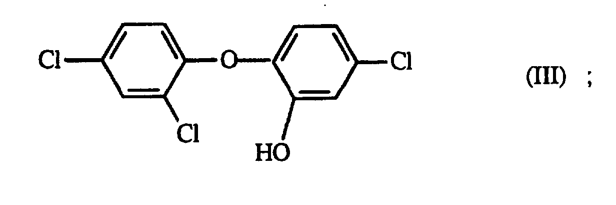
5) les composés azolés tels que le climbazole, le kétoconazole, le clotrinazole, l'econazole, l'isoconazole et le miconazole;
6) les polymères antifongiques tels que l'amphotéricine B ou la nystatine;
7) les sulfures de sélénium;
8) D'autres agents antipelliculaires sont le soufre sous ses différentes formes, le sulfure de cadmium, l'allantoïne, les goudrons de houille ou de bois et leurs dérivés en particulier l'huile de cade, l'acide undécylénique, l'acide fumarique, les allylamines telles que la terbinafine;
9) ou un mélange de ces agents antipelliculaires.

Les agents antipelliculaires mentionnés ci-dessus peuvent aussi le cas échéant être utilisés sous forme de leurs sels d'addition d'acides physiologiquement acceptables, notamment sous forme de sels d'acide sulfurique, nitrique, thiocyanique, chlorhydrique, bromhydrique, iodhydrique, phosphorique, acétique, benzoïque, glycolique, acéturique, succinique, nicotinique, tartrique, maléique, palmitique, méthane sulfonique, propanoïque, 2-oxopropanoïque, propanedioïque, 2-hydroxy-1,4-butanedioïque, 3-phényl-2-propènoïque, α-hydroxybenzèneacétique, éthanesulfonique, 2-hydroxyéthanesulfonique, 4-méthylbenzènesulfonique, 4-amino-2-hydroxybenzoïque, 2-phénoxybenzoïque, 2-acétyloxybenzoïque, picrique, lactique, citrique, malique, oxalique et d'aminoacides.

Les agents antipelliculaires mentionnés ci-dessus peuvent aussi le cas échéant être utilisés sous forme de leurs sels d'addition de bases organiques ou inorganiques physiologiquement acceptables. Des exemples de bases organiques sont notamment les alcanolamines de faibles poids moléculaires telles que l'éthanolamine, la diéthanolamine, la N-éthyléthanolamine, la triéthanolamine, le diéthylaminoéthanol, le 2-amino-2-méthylpropanedione; les bases non-volatiles telles que l'éthylènediamine, l'hexaméthylènediamine, la cyclohexylamine, la benzylamine, la N-méthylpipérazine; les hydroxydes d'ammonium quaternaires, par exemple le triméthylbenzyl hydroxyde; la guanidine et ses dérivés, et particulièrement ses dérivés alkylés. Des exemples de bases inorganiques sont notamment les sels de métaux alcalins, comme le sodium ou potassium; les sels d'ammonium, les sels de métaux alcalinoterreux, comme le magnésium, calcium; les sels de métaux di, tri ou tétravalents cationiques, comme le zinc, l'aluminium et le zirconium. Les alcanolamines, l'éthylènediamine et les bases inorganiques telles que les sels de métaux alcalins sont préférées.

Les agents antipelliculaires sont présents dans des proportions notamment comprises entre 0,001% et 10% en poids par rapport au poids total de la composition et préférentiellement dans des proportions comprises entre 0,01% et 5% en poids par rapport au poids total de la composition.

Les compositions selon l'invention contiennent de préférence en outre au moins un agent tensio-actif, notamment choisi parmi les tensio-actifs anioniques, amphotères, non-ioniques, cationiques et leurs mélanges.

Parmi les agents tensio-actifs anioniques, on peut citer les sels alcalins, les sels d'ammonium, les sels d'amines, les sels d'aminoalcools, les sels de magnésium des composés suivants : les alkylsulfates, alkyléthersulfates, alkylamidoéthersulfates, alkylarylpolyéthersulfates, monoglycérides sulfates; les alkylsulfonates, alkylamides sulfonates, alkylarylsulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylphosphates, alkyléther phosphates; les acylsarcosinates, les acyliséthionates et les N-acyltaurates.

Le radical alkyle ou acyle de ces différents composés est généralement constitué par une chaîne carbonée comportant de 8 à 30 atomes de carbone.

Parmi les agents tensio-actifs anioniques, on peut également citer les sels d'acides gras tels que les sels des acides oléique, ricinoléique, palmitique, stéarique; les acides d'huile de coprah ou d'huile de coprah hydrogénée; les acyl lactylates, dont le radical acyle comporte de 8 à 30 atomes de carbone.

On peut également utiliser des agents tensio-actifs considérés comme faiblement anioniques tels que les acides alkyl ou alkylaryl éthers carboxyliques polyoxyalkylénés ou leurs sels, les acides alkylamido éthers carboxyliques polyoxyalkylénés ou leurs sels, les acides d'alkyl D-galactoside uroniques ou leurs sels.

Les agents tensio-actifs non-ioniques sont plus particulièrement choisis parmi les alcools ou les alkylphénols ou les acides gras polyéthoxylés, polypropoxylés ou polyglycérolés, à chaîne grasse comportant 8 à 30 atomes de carbone, le nombre de groupements oxyde d'éthylène ou oxyde de propylène étant compris entre 2 et 50 et le nombre de groupements glycérol étant compris entre 2 et 30.

On peut également citer les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amides gras polyglycérolés comportant de préférence 1 à 5 groupements glycérol et en particulier 1,5 à 4; les amines grasses polyéthoxylées ayant de préférence 2 à 30 moles d'oxyde d'éthylène; les esters d'acides gras du sorbitan oxyéthylénés avec 2 à 30 moles d'oxyde d'éthylène; les esters d'acide gras de sucrose, les esters d'acides gras du polyéthylèneglycol, les alkylpolyglycosides, les dérivés carbamates ou amides de N-alkyl glucamines, les aldobionamides, les oxydes d'amines tels que les oxydes d'alkylamines ou de N-acylamidopropyl-morpholine.

Les agents tensio-actifs amphotères préférés sont les dérivés d'amines secondaires ou tertiaires aliphatiques, dans lesquels le radical aliphatique est une chaîne linéaire ou ramifiée comportant 8 à 22 atomes de carbone et qui contient au moins un groupe anionique hydrosolubilisant, carboxylate, sulfonate, sulfate, phosphate ou phosphonate; les alkyl(C₈₋₂₀)bétaïnes, les sulfobétaïnes, les alkyl(C₈-C₂₀)amidoalkyl(C₁-C₆) bétaïnes ou les alkyl(C₈-C₂)amidoalkyl(C₁-C₆)sulfobétaïnes.

Parmi les dérivés d'amines, on peut citer les produits vendus sous la dénomination MIRANOL, tels que décrits dans les brevets US-A-2 528 378 et 2 781 354 et classés dans le dictionnaire CTFA, 7ème édition, 1997, sous la dénomination Disodium Cocoamphodiacétate, Disodium Lauroamphodiacétate, Disodium Capryloamphodiacétate, Disodium Caproamphodiacétate, Disodium Cocoamphodipropionate, Disodium Lauroamphodipropionate, Disodium Caproamphodipropionate, Disodium Capryloamphodipropionate, Lauroamphodipropionate acide, Cocoamphodipropionate acide.

Les agents tensio-actifs cationiques sont notamment choisis parmi les sels d'amines grasses primaires, secondaires ou tertiaires, éventuellement polyoxyalkylénées; les sels d'ammonium quaternaire; les dérivés d'imidazoline; ou les oxydes d'amines à caractère cationique.

Les sels d'ammonium quaternaire préférés sont les halogénures (par ex. chlorures) de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore le chlorure de stéaramidopropyldiméthyl (myristyl acétate) ammonium commercialisé sous la dénomination «CEPHARYL 70» par la société VAN DYK.

On peut également utiliser les sels (chlorures ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol.

De préférence, on utilise au moins un tensioactif détergent, notamment si l'on désire obtenir des shampooings antipelliculaires.

Les agents tensio-actifs sont éventuellement utilisés dans les compositions conformes à l'invention dans des proportions de 0,01 à 50% en poids par rapport au poids total de la composition. Quand les compositions sont sous forme de shampooings, ils sont généralement utilisés à raison d'au moins 4% en poids, de préférence entre 5 et 50% en poids par rapport au poids total de la composition, et en particulier entre 8 et 35%.

Les compositions, selon l'invention, présentent un pH généralement compris entre 3 et 12, et plus particulièrement entre 4 et 8.

Le milieu cosmétiquement acceptable des compositions est constitué par de l'eau ou par un ou plusieurs solvants ou par un mélange d'eau et d'au moins un solvant cosmétiquement acceptable choisi parmi les alcools inférieurs, les alkylèneglycols et les éthers de polyol.

Dans un mode de réalisation préféré de l'invention, les compositions selon la présente invention contiennent des polyorganosiloxanes modifiés ou non, à savoir des huiles de polyorganosiloxanes ou des gommes ou des résines de polyorganosiloxanes, telles quelles ou sous forme de solutions dans des solvants organiques ou encore sous forme d'émulsions ou de microémulsions.

Parmi les polyorganosiloxanes pouvant être utilisés conformément à la présente invention, on peut citer à titre non limitatif :
I. Les silicones volatiles : celles-ci possèdent un point d'ébullition compris entre 60°C et 260°C. Elles sont choisies parmi les silicones cycliques contenant de 3 à 7 atomes de silicium et de préférence 4 à 5. Il s'agit, par exemple, de l'octaméthyl-cyclotétrasiloxane vendu sous le nom de "VOLATILE SILICONE 7207" par UNION CARBIDE ou "SILBIONE 70045 V2" par RHONE POULENC, le décaméthylcyclopentasiloxane vendu sous le nom de "VOLATILE SILICONE 7158" par UNION CARBIDE, "SILBIONE 70045 V5 par RHONE POULENC, ainsi que leurs mélanges. On cite également les cyclocopolymères du type diméthylsiloxane/méthylalkylsiloxane, tel que la "SILICONE VOLATILE FZ 3109" vendue par la Société UNION CARBIDE, qui est un cyclocopolymère diméthylsiloxane/méthyloctylsiloxane.
II. Les silicones non volatiles : elles sont constituées principalement par:
   (i) les polyalkylsiloxanes ; Parmi les polyalkylsiloxanes, on peut citer principalement les polydiméthylsiloxanes linéaires à groupements terminaux triméthylsilyle, comme par exemple, et à titre non limitatif, les huiles "SILBIONE" de la série 70047 commercialisées par RHODIA CHIMIE; les huiles DC 200 de DOW CORNING, les PDMS à groupements terminaux hydroxydiméthylsilyle ;
   (ii) les polyarylsiloxanes ;
   (iii) les polyalkylarylsiloxanes ; on peut citer les polyméthylphénylsiloxanes, les polydiméthylméthylphénylsiloxanes, les polydiméthyldiphénylsiloxanes linéaires et ramifiés, tels que par exemple l'huile "RHODORSIL 763" de RHODIA CHIMIE;
   (iv) les gommes de silicone ; ce sont des polydiorganosiloxanes de masse moléculaire comprise entre 200 000 et 5 000 000, utilisés seuls ou en mélange dans un solvant choisi parmi les silicones volatiles, les huiles polydiméthylsiloxanes (PDMS), les huiles polyphénylméthylsiloxanes, (PPMS), les isoparaffines, le chlorure de méthylène, le pentane, le dodécane, le tridécane, le tétradécane ou leurs mélanges; on cite, par exemple, les composés suivants :
      - polydiméthylsiloxane,
      - poly[(diméthylsiloxane)/(méthylvinylsiloxane)],
      - poly[(diméthylsiloxane)/(diphénylsiloxane)],
      - poly[(diméthylsiloxane))(phénylméthylsiloxane)],
      - poly[(diméthylsiloxane)/(diphénylsiloxane)/(méthylvinylsiloxane)];
   on peut aussi citer, par exemple, à titre non limitatif, les mélanges suivants :
1) les mélanges formés à partir d'un polydiméthylsiloxane hydroxylé en bout de chaîne (DIMETHICONOL selon la nomenclature CTFA), et d'un polydiméthylsiloxane cyclique (CYCLOMETHICONE selon la nomenclature CTFA), tels que le produit "Q2 1401" vendu par la Société DOW CORNING;
2) les mélanges formés à partir d'une gomme polydiméthylsiloxane avec une silicone cyclique, tel que le produit "SF 1214 SILICONE FLUID" de GENERAL ELECTRIC, qui est une gomme SE 30 de PM 500 000 solubilisée dans la "SF 1202 SILICONE FLUID" (décaméthylcyclopentasiloxane);
3) les mélanges de deux PDMS de viscosité différente, notamment d'une gomme PDMS et d'une huile PDMS, tels que les produits "SF 1236" et "CF 1241" de la Société GENERAL ELECTRIC;
   (v) les résines de silicone ; de préférence des systèmes siloxaniques réticulés renfermant les unités R₂SiO_{2/2}, RSiO_{3/2} et SiO_{4/2} dans lesquelles R représente un groupement hydrocarboné possédant 1 à 6 atomes de carbone ou un groupement phényle. Parmi ces résines, on peut citer le produit vendu sous la dénomination "DOW CORNING 593";
   (vi) les polyorganosiloxanes organomodifiés ; c'est-à-dire des silicones telles que définies précédemment, comportant dans leur structure générale, un ou plusieurs groupements organofonctionnels directement fixés sur la chaîne siloxanique ou fixés par l'intermédiaire d'un radical hydrocarboné; on cite, par exemple, les silicones comportant :
      a) des groupements polyéthylèneoxy et/ou polypropylèneoxy, comportant éventuellement des groupes alkyle, tels que le produit dénommé diméthicone copolyol vendu par la Société DOW CORNING sous la dénomination "DC 1248", et l'alkyl (C12) méthicone copolyol vendu par la Société DOW CORNING sous la dénomination "Q2 5200";
      b) des groupements (per)fluorés comme les groupements trifluoroalkyle, telles que, par exemple, celles vendues par la Société GENERAL ELECTRIC sous les dénominations "FF.150 Fluorosilicone Fluid";
      c) des groupements hydroxyacylamino, telles que celles décrites dans la demande de brevet européen EP-A-0 342 834 et en particulier la silicone vendue par la Société DOW CORNING sous la dénomination "Q2-8413" ;
      d) des groupements thiols comme dans les silicones "X 2-8360" de DOW CORNING ou les "GP 72A" et "GP 71" de GENESEE ;
      e) des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ou aminoalkyl(C₁-C₄)aminoalkyl(C₁-C₄). On utilise plus particulièrement les silicones dénommées amodiméthicone et triméthylsilylamodiméthicone selon la dénomination CTFA (1997) ;
      f) des groupements carboxylates, comme les produits décrits dans le brevet européen EP 186 507 de CHISSO CORPORATION;
      g) des groupements hydroxylés, comme les polyorganosiloxanes à fonction hydroxyalkyle, décrits dans la demande de brevet FR-A-2 589 476 ;
      h) des groupements alcoxylés comportant au moins 12 atomes de carbone comme le produit "SILICONE COPOLYMER F 755" de SWS SILICONES;
      i) des groupements acyloxyalkyle comportant au moins 12 atomes de carbone, comme par exemple les polyorganosiloxanes décrits dans la demande de brevet FR-A-2 641 185;
      j) des groupements ammonium quaternaire, comme dans le produit "ABIL K 3270" de la Société GOLDSCHMIDT ;
      k) des groupements amphotères ou bétaïniques, tels que dans le produit vendu par la Société GOLDSCHMIDT sous la dénomination "ABIL B 9950" ;
      l) des groupements bisulfite, tels que dans les produits vendus par la Société GOLDSCHMIDT sous les dénominations "ABIL S 201 " et "ABIL S 255";
   (vii) les copolymères blocs ayant un bloc linéaire polysiloxane-polyalkylène comme unité répétitive ; la préparation de tels copolymères blocs mis en oeuvre dans le cadre de la présente invention est décrite dans la demande européenne EP 0 492 657 A1;
   (viii) les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ; notamment ceux choisis plus préférentiellement parmi ceux décrits dans les brevets US 4.963.935, US 4.728.571 et US 4.972.037 et les demandes de brevet EP-A 0 412 704, EP-A-0 412 707, EP-A-0 640 105 et WO 95/00578;
   (ix) les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ; des exemples de tels polymères, ainsi que leur mode particulier de préparation, sont notamment décrits dans les demandes de brevets EP-A-0 582 152, WO 93/23009 et WO 95/03776;
   (x) ou leurs mélanges.

Les polyorganosiloxanes préférés pour être utilisés selon l'invention sont les polyorganopolysiloxanes non volatils et préférentiellement les huiles ou gommes polydiméthylsiloxanes éventuellement aminés, arylés ou alkylarylés.

Les polyorganosiloxanes sont utilisés dans les compositions de l'invention dans des proportions comprises entre 0,01 et 20% en poids et de préférence entre 0,1 et 10% en poids par rapport au poids total de la composition.

Dans un autre mode de réalisation préféré, les compositions de l'invention contiennent en outre au moins un polymère cationique choisi parmi tous ceux déjà connus en soi, notamment ceux décrits dans la demande de brevet EP-A-0 337 354 et dans les demandes de brevets français FR-A-2 270 846, 2 383 660, 2 598 611, 2 470 596 et 2 519 863.

Les polymères cationiques utilisés ont généralement une masse moléculaire comprise entre 500 et 5.10⁶ environ, et de préférence comprise entre 10³ et 3.10⁶ environ .

Parmi les polymères cationiques, on peut citer les protéines (ou hydrolysats de protéines) quaternisées et les polymères du type polyamine, polyaminoamide et polyammonium quaternaire. Ce sont des produits connus.

Les protéines ou hydrolysats de protéines quaternisés sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :
- les hydrolysats de collagène portant des groupements triéthylammonium tels que les produits dénommés dans le dictionnaire CTFA "TRIETHONIUM HYDROLYZED COLLAGEN ETHOSULFATE" ;
- les hydrolysats de collagène portant des groupements chlorure de triméthylammonium et de triméthylstéarylammonium, dénommés dans le dictionnaire CTFA "STEARTRIMONIUM HYDROLYZED COLLAGEN" ;
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres le "CROQUAT L", le "CROQUAT M", le "CROQUAT S" et le "CROTEIN Q" vendus par la Société CRODA.

D'autre protéines ou hydrolysats quaternisés sont par exemple ceux vendus par la Société INOLEX, sous la dénomination "LEXEIN QX 3000".

On peut encore citer les protéines végétales quaternisées telles que les protéines de blé, de maïs ou de soja: comme protéines de blé quaternisées, on peut citer celles appelées dans le dictionnaire CTFA "COCODIMONIUM HYDROLYSED WHEAT PROTEIN", "LAURIDIMONIUM HYDROLYSED WHEAT PROTEIN", ou encore "STEARDIMONIUMHYDROLYSED WHEATPROTEIN".

Les polymères du type polyamine, polyaminoamide, polyammonium quaternaire, utilisables conformément à la présente invention, pouvant être notamment mentionnés, sont ceux décrits dans les brevets français n°2 505 348 ou 2 542 997. Parmi ces polymères, on peut citer:
(1) Les copolymères vinylpyrrolidone-acrylate ou - méthacrylate de dialkylaminoalkyle quaternisés ou non, tels que les polymères décrits en détail dans les brevets français 2 077 143 et 2 393 573.
(2) Les dérivés d'éthers de cellulose comportant des groupements ammonium quaternaires décrits dans le brevet français 1 492 597.
(3) Les dérivés de cellulose cationiques tels que les copolymères de cellulose ou les dérivés de cellulose greffés avec un monomère hydrosoluble d'ammonium quaternaire, et décrits notamment dans le brevet US 4131 576, tels que les hydroxyalkyl celluloses, comme les hydroxyméthyl-, hydroxyéthyl- ou hydroxypropyl celluloses greffées notamment avec un sel de méthacryloyléthyl triméthyl ammonium, de méthacrylamidopropyl triméthyl ammonium ou de diméthyldiallylammonium.
(4) Les polysaccharides et notamment gommes de guar cationiques décrits plus particulièrement dans les brevets US 3 589 578 et 4 031 307.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé, l'agent réticulant étant utilisé dans des proportions allant de 0,025 à 0,35 mole par groupement amine du polyaminoamide; ces polyaminoamides peuvent être alcoylés ou s'ils comportent une ou plusieurs fonctions amines tertiaires, quaternisés. De tels polymères sont notamment décrits dans les brevets français 2.252.840 et 2.368.508.
(7) Les dérivés de polyaminoamides résultant de la condensation de polyalcoylène polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone, le rapport molaire entre la polyalkylène polyamine et l'acide dicarboxylique étant compris entre 0,8 : 1 et 1,4 : 1, le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
(9) Les cyclopolymères de méthyl diallyl amine ou de diméthyl diallyl ammonium, notamment ceux décrits dans le brevet français 2.080.759 et dans son certificat d'addition 2.190.406.
(10) Les polymères de diammonium quaternaire décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
(11) Les polymères de polyammonium quaternaire notamment décrits dans la demande de brevet EP-A-122 324.
(12) Les homopolymères ou copolymères dérivés des acides acrylique ou méthacrylique et comportant des motifs CH₂-CHRₐ-CO-O-A₁-NRₑR_{f} , CH₂-CHRₐ-CO-O-A₁-N⁺R_{b}R_{c}R_{d}, X⁻ et/ou CH₂-CHRₐ-CO-NH-A₁-N⁺R_{b}R_{c}R_{d} X⁻
   dans lesquels les groupements Rₐ désignent indépendamment H ou CH₃, les groupements A₁ désignent indépendamment un groupe alcoyle linéaire ou ramifié de 1 à 6 atomes de carbone ou un groupe hydroxyalcoyle de 1 à 4 atomes de carbone, les groupements R_{b}, R_{c}, R_{d}, identiques ou différents, désignent indépendamment un groupe alcoyle de 1 à 18 atomes de carbone ou un radical benzyle, les groupements Rₑ et R_{f} représentent un atome d'hydrogène ou un groupement alcoyle de 1 à 6 atomes de carbone, X⁻ désigne un anion, par exemple méthosulfate ou halogénure, tel que chlorure ou bromure,
(13) Les polymères quaternaires de vinylpyrrolidone et de vinylimidazole tels que par exemple les produits commercialisés sous les dénominations "LUVIQUAT FC 905", "LUVIQUAT FC 550" et "LUVIQUAT FC 370" par la société BASF.
(14) Les polyamines comme le "POLYQUART H" vendu par HENKEL, référencé sous le nom de « POLYETHYLENEGLYCOL TALLOW POLYAMINE » dans le dictionnaire CTFA.
(15) Les polymères réticulés de sels de méthacryloyloxyéthyl triméthylammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère les dérivés d'éther de cellulose comportant des groupements ammonium quaternaires, les polysaccharides et notamment gomme de guar cationiques et les cyclopolymères de méthyl diallyl amine ou de diméthyl diallyl ammonium.

Les polymères cationiques sont utilisés dans les compositions de l'invention dans des proportions comprises entre 0,001 et 20% en poids et de préférence entre 0,05 et 5% en poids par rapport au poids total de la composition.

Les compositions selon l'invention peuvent également contenir en outre au moins un adjuvant choisi parmi les adjuvants habituellement utilisés en cosmétique, tel que les parfums, les conservateurs, les séquestrants, les humectants, les sucres, les huiles végétales, animales, minérales ou de synthèse, les polymères amphotères, le menthol, les dérivés de nicotinate, les agents anti-chute des cheveux, les stabilisateurs de mousse, les agents propulseurs, les colorants, les céramides, les vitamines ou provitamines, les agents acidifiants ou alcalinisants ou d'autres adjuvants cosmétiques bien connus.

Dans une forme de réalisation préférée de l'invention, les compositions selon l'invention sont utilisées comme shampooings pour le lavage et le traitement des cheveux et du cuir chevelu. Elles peuvent aussi être utilisées comme lotions antipelliculaires ou après-shampooing antipelliculaires.

Le procédé de traitement des cheveux consiste à appliquer une composition telle que définie ci-dessus sur des cheveux humides ou secs dans des quantités efficaces, cette application étant suivie facultativement d'un rinçage après un temps de pose facultatif.

Les exemples qui suivent sont destinés à illustrer l'invention.

| **EXEMPLE I : SHAMPOOING ANTIPELLICULAIRE** | |
|---|---|
| Distéarate de glycol | 2 g |
| N-cocoyl amidoéthyl, N-éthoxycarboxymethyl glycinate de sodium à 38% M.A vendu sous la dénomination "MIRANOL C2M conc" par la société RHODIA CHIMIE. | 2 g |
| Cocoyl bétaïne en solution aqueuse à 30% vendue sous la dénomination "DEHYTON AB 30" par la société HENKEL | 4 g |
| Composé vendu sous la dénomination "Octopirox" par la société HOECHST | 2 g |
| Hydroxyéthyl cellulose réticulée à l'épichlorhydrine, quaternisée par la triméthylamine, vendue sous la dénomination JR 400 par la société UNION CARBIDE | 0,25 g |
| Lauryl éther sulfate de sodium (2,2 OE) à 70% de M.A. | 18 g |
| Terpolymère d'acrylates, d'amino(méth)acrylates et d'itaconate d'alkyle en C₁₀-C₃₀ polyoxyéthyléné à 20 moles d'oxyde d'éthylène sous forme de dispersion aqueuse à 20% de M.A. vendu par la société National Starch sous la dénomination "STRUCTURE® PLUS" | 1,5 g |
| Acide citrique | 2 g |
| Conservateurs, parfum qs | |
| Eau déminéralisée stérilisée qsp | 100 g |

On ajuste le pH à 5,5 par de l'hydroxyde de sodium.

Après utilisation de ce shampooing, les cheveux sensibilisés mouillés sont doux, souples et les cheveux séchés sont lisses et doux et se démêlent facilement. L'efficacité antipelliculaire est bonne.

| **EXEMPLE II : SHAMPOOING ANTIPELLICULAIRE** | |
|---|---|
| Chlorure de sodium | 0,3 g |
| Vitamine B3 ou PP : amide nicotinique | qs |
| N-cocoyl amidoéthyl, N-éthoxycarboxyméthyl glycinate de sodium (38 %) | 1,5 g |
| Vitamine B6 : chlorhydrate de pyridoxine | qs |
| Chlorure d'hydroxypropyl guar triméthyl ammonium vendu sous le dénomination "JAGUAR C 13S" par la société MEYHALL | 0,04 g |
| 1-hydroxy-4-méthyl-6-(2,4,4-triméthylpentyl) -2-pyridone, sel de monoéthanolamine vendu sous la dénomination "Octopirox" par la société HOECHST | 0,5 g |
| Polydiméthylsiloxane de viscosité 0,3 m².s⁻¹ vendu sous le dénomination DC 200/300.000 par la société DOW CORNING | 1,8 g |
| Alcool laurique oxyéthyléné (2,5 OE) | 0,75 g |
| Extrait de fruits en solution aqueuse | qs |
| Monoisopropanolamide d'acides de coprah | 2 g |
| Cocoyl amidopropyl betaïne en solution aqueuse à 38% | 2,7 g |
| Lauryl éther sulfate de sodium (2,2 OE) à 70% | 17 g |
| PYRUS MALUS (INCI) | qs |
| Distéaryl éther | 1,5 g |
| Terpolymère d'acrylates, d'amino(méth)acrylates et d'itaconate d'alkyle en C₁₀-C₃₀ polyoxyéthyléné à 20 moles d'oxyde d'éthylène en dispersion aqueuse à 20% M.A. vendu sous la dénomination "STRUCTURE® PLUS" par la Société National Starch | 1 g |
| Mélange d'alcools linéaires(C18/C20/C22) | 1,5 g |
| Parfum, conservateurs qs | |
| Eau déminéralisée stérilisée qsp | 100 g |

On ajuste le pH à 7,5 par de l'acide citrique ou de l'hydroxyde de sodium. Après utilisation de ce shampooing, les cheveux sensibilisés mouillés sont doux, souples et les cheveux séchés sont lisses et doux et se démêlent facilement. L'efficacité antipelliculaire est bonne.

| **EXEMPLE III : SHAMPOOING ANTIPELLICULAIRE** | |
|---|---|
| Lauryl éther sulfate de sodium (2,2 OE) à 70% M.A. | 16 g |
| Cocoyl bétaïne en solution aqueuse à 30% commercialisée sous la dénomination "DEHYTON AB 30" par la société HENKEL | 6 g |
| Terpolymère d'acrylates, d'amino(méth)acrylates et d'itaconate d'alkyle en C₁₀-C₃₀ polyoxyéthyléné à 20 moles d'oxyde d'éthylène sous forme de dispersion aqueuse à 20% de M.A. vendu par la société National Starch sous la dénomination "STRUCTURE ® PLUS" | 1,5 g |
| Monoisopropanolamide d'acides de coprah | 1 g |
| Disulfure de sélenium en poudre micronisée commercialisé sous la dénomination "SELENIUM SULPHIDE USP MICRONISED" par la société FERRO METAL | 0.5 g |
| Conservateurs qs | |
| Eau déminéralisée stérilisée qsp | 100 g |

On ajuste le pH à 6,5 par de l'acide citrique ou de l'hydroxyde de sodium.

Après utilisation de ce shampooing, les cheveux sensibilisés mouillés sont doux, souples et les cheveux séchés sont lisses et doux et se démêlent facilement. L'efficacité antipelliculaire est bonne.

## Revendications

1. Composition de traitement antipelliculaire des cheveux et du cuir chevelu, comprenant dans un milieu cosmétiquement acceptable au moins un agent antipelliculaire, **caractérisée par le fait qu'**elle comprend en outre au moins un terpolymère acrylique constitué:
- de 5 à 80% en poids, préférentiellement de 15 à 70% en poids et plus préférentiellement de 40 à 70% en poids, d'un monomère acrylate (a) choisi parmi un acrylate d'alkyle en C₁-C₆ et un méthacrylate d'alkyle en C₁-C₆;
- de 5 à 80% en poids, préférentiellement de 10 à 70% en poids et plus préférentiellement de 20 à 60% en poids, d'un monomère (b) choisi parmi un composé vinylique héterocyclique contenant au moins un atome d'azote ou de soufre, un (méth)acrylamide, un (méth)acrylate de mono- ou di-(C₁-C₄)alkylamino(C₁-C₄)alkyle et un mono ou di (C₁-C₄)alkylamino (C₁-C₄) alkyl (méth)acrylamide;
- de 0,1 à 30% en poids, préférentiellement de 0,1 à 10% en poids d'un monomère (c) choisi parmi :
un uréthane produit par réaction entre un isocyanate insaturé monoéthylénique et un agent tensioactif non ionique englobant un copolymère séquencé d'oxyde de 1,2-butylène et d'oxyde d'éthylène à extrémité alcoxy en C₁₋₄ ;
un monomère tensioactif insaturé éthylénique copolymérisable obtenu par condensation d'un tensioactif non-ionique avec un acide carboxylique insaturé α,β-éthylénique ou son anhydride;
un monomère tensioactif choisi parmi les produits de réaction de type urée d'un monoisocyanate insaturé monoéthylénique avec un tensioactif non ionique présentant une fonctionalité amine;
un éther de (méth)allyle de formule CH₂=CR₁CH₂OAₘBₙAₚR₂ dans lequel R₁ désigne un atome d'hydrogène ou un groupe méthyle, A désigne un groupement propylèneoxy ou butylèneoxy, B désigne éthylèneoxy, n est égal à zéro ou désigne un nombre entier inférieur ou égal à 200, m et p désignent zéro ou un nombre entier inférieur à n et R₂ est un groupe hydrophobe d'au moins 8 atomes de carbone; et
un monomère non-ionique de type uréthane produit par réaction d'un tensioactif non ionique monohydrique avec un isocyanate insaturé monoéthylénique;
et éventuellement d'un monomère de réticulation
les pourcentages en poids de monomères étant basés sur le poids total des monomères constituant le terpolymère,
et en ce que l'agent antipelliculaire est choisi parmi:
1) le chlorure de benzéthonium, le chlorure de benzalkonium, la chlorhexidine, la chloramine T, la chloramine B, la 1,3-dibromo 5,5-diméthylhydantoïne, la 1,3-dichloro 5,5-diméthylhydantoïne, la 3-bromo 1-chloro 5,5-diméthylhydantoïne, le N-chlorosuccinimide;
2) les dérivés de 1-hydroxy-2-pyridone représentés par la formule (I): 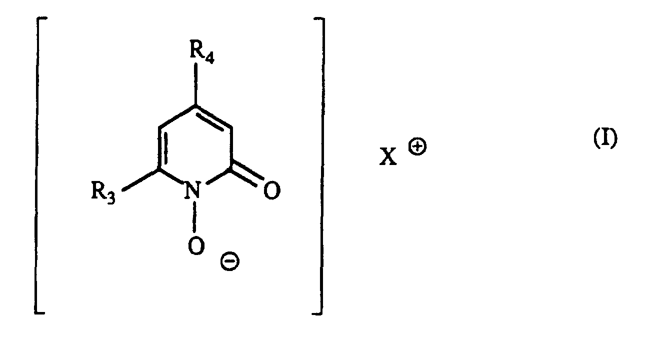 dans laquelle R₃ représente un groupe alkyle ayant de 1 à 17 atomes de carbone, alkényle ayant de 2 à 17 atomes de carbone, un groupe cycloalkyle ayant de 5 à 8 atomes de carbone, un groupe bicycloalkyle ayant de 7 à 9 atomes de carbone; un groupe cycloalkyl (-alkyle), un groupe aryle, un groupe aralkyle avec un alkyle ayant de 1 à 4 atomes de carbone, un groupe arylalkényl avec un alkényle ayant de 2 à 4 atomes de carbone, aryloxyalkyle ou arylmercaptoalkyle avec un alkyle ayant de 1 à 4 atomes de carbone, un groupe furylalkényl avec un alkényle ou un furyle ayant de 2 à 4 atomes de carbone, un groupe alkoxy ayant de 1 à 4 atomes de carbone, un groupe nitro, un groupe cyano ou un atome d'halogène;
R₄ représente un atome d'hydrogène, un groupe alkyle en C₁-C₄, un groupe alkényle en C₂-C₄, un atome d'halogène, un groupe phényle, un groupe benzyle; X représente une base organique, un ion de métal alcalin ou alcalinoterreux, un ion ammonium; et ces dérivés sous forme de sels avec des bases organiques ou inorganiques.
3) les trihalogéno carbamides de formule (II) suivante: 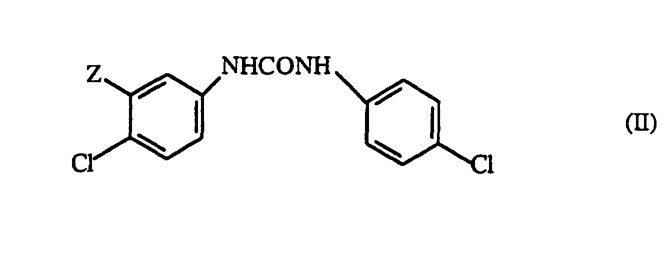 dans laquelle Z représente un atome d'halogène comme le chlore ou un groupement trihalogénoalkyle en C₁-C₄ tel que CF3;
4) le triclosan représenté par la formule (III) 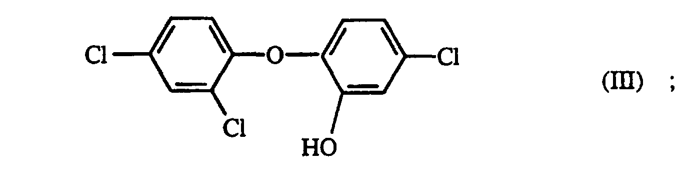
5) les composés azolés tels que le climbazole, le kétoconazole, le clotrinazole, l'econazole, l'isoconazole et le miconazole;
6) les polymères antifongiques tels que l'amphotéricine B ou la nystatine;
7) les sulfures de sélénium;
8) le soufre sous ses différentes formes, le sulfure de cadmium, l'allantoïne, les goudrons de houille ou de bois, l'acide undécylénique, l'acide fumarique et les allylamines;
9) un mélange de ces agents antipelliculaires.

2. Composition selon la revendication 1, **caractérisée en ce que** le terpolymère est présent à raison de 0,01 à 20% en poids de matière active, de préférence 0,1 à 10 % en poids par rapport au poids total de la composition.

3. Composition selon la revendication 1 ou 2, **caractérisée en ce que** le monomère (a) est choisi parmi les acrylates d'alkyle en C₂-C₆, et est préférentiellement l'acrylate d'éthyle.

4. Composition selon l'une quelconque des revendications 1 à 3, **caractérisée en ce que** le monomère (b) est choisi parmi le méthacrylate de N,N-diméthylaminoéthyle, l'acrylate de N,N-diéthylaminoéthyle, le méthacrylate de N,N-diéthylaminoéthyle, l'acrylate de N-t-butylaminoéthyle, le méthacrylate de N-t-butylaminoéthyle, le N,N-diméthylaminopropyl-acrylamide, le N,N-diméthylaminopropyl-méthacrylamide, le N,N-diéthylaminopropyl-acrylamide et le N,N-diéthylaminopropyl-méthacrylamide, et est préférentiellement le méthacrylate de N,N-diméthylaminoéthyle.

5. Composition selon l'une quelconque des revendications 1 à 4, **caractérisée en ce que** le monomère (c) est un monomère tensioactif insaturé éthylénique copolymérisable obtenu par condensation d'un tensioactif non-ionique avec l'acide itaconique.

6. Composition selon l'une quelconque des revendications 1 à 5, **caractérisée en ce que** le terpolymère acrylique est constitué d'acrylates, d'amino(méth)acrylates et d'itaconate d'alkyle en C₁₀-C₃₀ polyoxyéthyléné à 20 moles d'oxyde d'éthylène.

7. Composition selon l'une quelconque des revendications 1 à 6, **caractérisée en ce que** le terpolymère acrylique contient en outre un monomère de réticulation.

8. Composition selon l'une quelconque des revendications 1 à 7, **caractérisée par le fait que** l'agent antipelliculaire est présent à raison de 0,001 à 10% en poids, de préférence de 0,01 à 5 % en poids par rapport au poids total de la composition.

9. Composition selon l'une quelconque des revendications 1 à 8, **caractérisée en ce qu'**elle contient en outre au moins un agent tensio-actif choisi parmi les agents tensio-actifs anioniques, amphotères, non-ioniques, cationiques et leurs mélanges.

10. Composition selon la revendication 9, **caractérisée par le fait que** les agents tensio-actifs anioniques sont choisis parmi les sels alcalins, les sels de magnésium, les sels d'ammonium, les sels d'amines ou les sels d'aminoalcools des composés suivants : les alkylsulfates, alkyléthersulfates, alkylamidoéthersulfates, alkylarylpolyéthersulfates, monoglycérides sulfates; les alkylsulfonates, alkylamides sulfonates, alkylaryl-sulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylphosphates, alkyléther phosphates; les acylsarcosinates, les acyliséthionates, N-acyltaurates; le radical alkyle ou acyle de ces différents composés étant constitué par une chaîne carbonée comportant de 8 à 30 atomes de carbone; les sels d'acides gras des acides oléique, ricinoléique, palmitique, stéarique; les acides d'huile de coprah ou d'huile de coprah hydrogénée; les acyl lactylates, dont le radical acyle comporte de 8 à 30 atomes de carbone; les acides d'alkyl D-galactoside uroniques et leurs sels, les acides alkyl ou alkylaryl éthers carboxyliques polyoxyalkylénés ou leurs sels, les acides alkylamido éthers carboxyliques polyoxyalkylénés ou leurs sels.

11. Composition selon la revendication 9, **caractérisée par le fait que** les agents tensio-actifs non-ioniques sont choisis parmi les alcools ou les alkylphénols ou les acides gras polyéthoxylés, polyoxypropylénés ou polyglycérolés, à chaîne grasse comportant 8 à 30 atomes de carbone, le nombre de groupements oxyde d'éthylène, oxyde de propylène étant compris entre 2 et 50 et le nombre de groupements glycérol étant compris entre 2 et 30; les copolymères d'oxyde d'éthylène et de propylène; les condensats d'oxyde d'éthylène et de propylène sur des alcools gras; les amides gras polyéthoxylés; les amides gras polyglycérolés; les amines grasses polyéthoxylées; les esters d'acides gras du sorbitan oxyéthylénés; les esters d'acides gras de sucrose ou du polyéthylèneglycol; les alkylpolyglycosides; les dérivés amides ou carbamates de N-alkylglucamines, les aldobionamides et les oxydes d'amines.

12. Composition selon la revendication 9, **caractérisée par le fait que** les agents tensio-actifs amphotères sont choisis parmi les dérivés d'amines secondaires ou tertiaires aliphatiques, dans lesquels le radical aliphatique est une chaîne linéaire ou ramifiée comportant de 8 à 22 atomes de carbone et qui contient au moins un groupe anionique hydrosolubilisant, carboxylate, sulfonate, sulfate, phosphate, phosphonate; les alkyl(C₈-C₂₀)bétaïnes, les sulfobétaïnes, les alkyl(C₈-C₂₀)amidoalkyl(C₁-C₆) bétaïnes ou les alkyl(C₈-C₂₀)amidoalkyl(C₁-C₆)sulfobétaïnes.

13. Composition selon la revendication 9, **caractérisée par le fait que** les agents tensio-actifs cationiques sont choisis parmi les sels d'amines grasses primaires, secondaires ou tertiaires, éventuellement polyoxyalkylénées; les sels d'ammonium quaternaire; les dérivés d'imidazoline; et les oxydes d'amines à caractère cationique.

14. Composition selon la revendication 9, **caractérisée en ce que** l'agent tensio-actif est présent à raison de 0,01% à 50% en poids par rapport au poids total de la composition.

15. Composition selon la revendication 14, **caractérisée en ce que** l'agent tensio-actif est présent à raison d'au moins 4% en poids, de préférence de 5 à 50% en poids, et encore plus préférentiellement de 8 à 35% en poids par rapport au poids total de la composition.

16. Composition selon l'une quelconque des revendications 1 à 15, **caractérisée en ce qu'**elle présente un pH compris entre 3 et 12, et plus particulièrement entre 4 et 8.

17. Composition selon l'une quelconque des revendications 1 à 16, **caractérisée par le fait que** le milieu cosmétiquement acceptable est constitué par de l'eau ou par un ou plusieurs solvants ou par un mélange d'eau et d'au moins un solvant cosmétiquement acceptable choisi parmi les alcools inférieurs, les alkylèneglycols et les éthers de polyol.

18. Composition selon l'une quelconque des revendications 1 à 17, **caractérisée par le fait qu'**elle contient en outre au moins un polyorganosiloxane choisi parmi les silicones volatiles et les silicones non-volatiles et notamment
(i) les polyalkylsiloxanes ;
(ii) les polyarylsiloxanes ;
(iii) les polyalkylarylsiloxanes ;
(iv) les gommes de silicone ;
(v) les résines de silicone ;
(vi) les polyorganosiloxanes organomodifiés ;
(vii) les copolymères blocs ayant un bloc linéaire polysiloxane-polyalkylène comme unité répétitive ;
(viii) les polymères siliconés greffés, à squelette organique non siliconé;
(ix) les polymères siliconés greffés, à squelette polysiloxanique greffé par des monomères organiques non siliconés;
(x) et leurs mélanges.

19. Composition selon la revendication 18, **caractérisée par le fait qu'**elle contient un polyorganosiloxane dans des proportions comprises entre 0,01 et 20% en poids et de préférence entre 0,1 et 10% en poids par rapport au poids total de la composition.

20. Composition selon l'une quelconque des revendications 1 à 19, **caractérisée par le fait qu'**elle contient en outre au moins un polymère cationique choisi parmi :
- les protéines ou hydrolysats de protéines, en particulier les hydrolysats de collagène portant des groupements triéthylammonium, les hydrolysats de collagène portant des groupements chlorure de triméthylammonium et de triméthylstéarylammonium, les hydrolysats de protéines portant sur la chaîne polypeptidique des groupements ammonium quaternaire comportant au moins un radical alkyle ayant de 1 à 18 atomes de carbone, les protéines végétales quaternisées telles que les protéines de blé, de maïs ou de soja;
- les polymères du type polyamine, polyaminoamide, polyammonium quaternaire, en particulier
i) les copolymères vinylpyrrolidone-acrylate ou méthacrylate de dialkylaminoalkyle quaternisés ou non,
ii) les dérivés d'éthers de cellulose comportant des groupements ammonium quaternaires,
iii) les dérivés de cellulose cationiques tels que les copolymères de cellulose ou les dérivés de cellulose greffés avec un monomère hydrosoluble d'ammonium quaternaire,
iv) les polysaccharides et notamment gommes de guar cationiques,
v) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères,
vi) les polyaminoamides solubles dans l'eau préparés en particulier par polycondensation d'un composé acide avec une polyamine,
vii) les dérivés de polyaminoamides résultant de la condensation de polyalcoylène polyamines avec des acides polycarboxyliques suivie d'une alcoylation par des agents bifonctionnels,
viii) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone,
ix) les cyclopolymères de méthyl diallyl amine ou de diméthyl diallyl ammonium,
x) les polymères de diammonium quaternaire,
xi) les polymères de polyammonium quaternaire,
xii) les homopolymères ou copolymères dérivés des acides acrylique ou méthacrylique et comportant des motifs CH₂-CHRₐ-CO-O-A₁-NRₑR_{f}, CH₂-CHRₐ-CO-O-A₁-N⁺R_{b}R_{c}R_{d}, X⁻ et/ou CH₂-CHRₐ-CO-NH-A₁-N⁺R_{b}R_{c}R_{d}, X⁻
dans lesquels les groupements Rₐ désignent indépendamment H ou CH₃, les groupements A₁ désignent indépendamment un groupe alcoyle linéaire ou ramifié de 1 à 6 atomes de carbone ou un groupe hydroxyalcoyle de 1 à 4 atomes de carbone, les groupements R_{b}, R_{c}, R_{d}, identiques ou différents, désignent indépendamment un groupe alcoyle de 1 à 18 atomes de carbone ou un radical benzyle, les groupements Rₑ et R_{f} représentent un atome d'hydrogène ou un groupement alcoyle de 1 à 6 atomes de carbone, X⁻ désigne un anion, par exemple méthosulfate ou halogénure, tel que chlorure ou bromure,
xiii) les polymères quaternaires de vinylpyrrolidone et de vinylimidazole,
xiv) les polyamines référencé sous le nom de « POLYETHYLENEGLYCOL TALLOW POLYAMINE » dans le dictionnaire CTFA,
xv) les polymères réticulés de chlorure de méthacryloyloxyéthyl triméthylammonium,
- les polyalkylèneimines, les polymères contenant des motifs vinylpyridine ou vinylpyridinium, les condensats de polyamines et d'épichlorhydrine, les polyuréylènes quaternaires et les dérivés de la chitine.

21. Composition selon la revendication 20, **caractérisée par le fait qu'**elle contient un polymère cationique dans des proportions comprises entre 0,001 et 20% en poids et de préférence entre 0,05 et 5% en poids par rapport au poids total de la composition.

22. Composition selon l'une quelconque des revendications 1 à 20, **caractérisée par le fait qu'**elle contient en outre au moins un adjuvant cosmétiquement acceptable choisi parmi les parfums, les conservateurs, les filtres, les séquestrants, les humectants, les sucres, les huiles végétales, animales, minérales ou de synthèse, les polymères amphotères, le menthol, les dérivés de nicotinates, les agents antichute des cheveux, les stabilisateurs de mousse, les agents propulseurs, les colorants, les céramides, les vitamines ou provitamines et les agents acidifiants ou alcalinisants.

23. Utilisation cosmétique comme shampooing, lotion ou après-shampooing d'une composition telle que définie dans l'une quelconque des revendications 1 à 22.

24. Procédé de traitement cosmétique des cheveux, **caractérisé par le fait que** l'on applique sur les cheveux humides ou secs au moins une composition telle que définie dans l'une quelconque des revendications 1 à 22, et facultativement, après un temps de pose facultatif, on les rince à l'eau.

## Patentansprüche

1. Zusammensetzung zur Behandlung der Haare und der Kopfhaut gegen Schuppen, die in einem kosmetisch akzeptablen Medium mindestens ein Antischuppenmittel enthält, **dadurch gekennzeichnet, dass** sie ferner mindestens ein Acrylterpolymer enthält, das besteht aus:
- 5 bis 80 Gew.-%, vorzugsweise 15 bis 70 Gew.-% und noch bevorzugter 40 bis 70 Gew.-% eines Acrylatmonomers (a), das unter den C₁₋₆-Alkylacrylaten und C₁₋₆-Alkylmethacrylaten ausgewählt ist;
- 5 bis 80 Gew.-%, vorzugsweise 10 bis 70 Gew.-% und noch bevorzugter 20 bis 60 Gew.-% eines Monomers (b), das unter den heterocyclischen Vinylverbindungen, die mindestens ein Stickstoffatom oder Schwefelatom enthalten, (Meth)acrylamiden, Mono- oder Di-C₁₋₄-alkylamino-C₁₋₄-alkyl(meth)-acrylaten und Mono- oder Di-C₁₋₄-Alkylamino-C₁₋₄-alkyl(meth)-acrylamiden ausgewählt ist;
- 0,1 bis 30 Gew.-% und vorzugsweise 0,1 bis 10 Gew.-% eines Monomers (c), das ausgewählt ist unter:
· Urethanen, die bei der Umsetzung eines monoethylenisch ungesättigten Isocyanats mit einem nichtionischen grenzflächenaktiven Stoff, der ein Sequenzcopolymer von 1,2-Butylenoxid und Ethylenoxid mit endständiger C₁₋₄-Alkoxygruppe einschließt, entsteht;
· copolymerisierbaren, ethylenisch ungesättigten Tensidmonomeren, die bei der Kondensation von nichtionischen grenzflächenaktiven Stoffen mit α,β-ethylenisch ungesättigten Carbonsäuren oder ihren Anhydriden gebildet werden;
· Tensidmonomeren, die unter den Reaktionsprodukten vom Harnstofftyp von monoethylenisch ungesättigten Monoisocyanaten mit nichtionischen grenzflächenaktiven Stoffen, die eine Aminofunktion aufweisen, ausgewählt sind;
· (Meth)allylethern der Formel CH₂=CR₁CH₂OAₘBₙAₚR₂, wobei in der Formel R₁ ein Wasserstoffatom oder die Methylgruppe bedeutet, A eine Propylenoxy- oder Butylenoxygruppe bezeichnet, B Ethylenoxy ist, n Null oder eine ganze Zahl von höchstens 200 und m und p Null oder eine ganze Zahl unter n bedeuten und R₂ eine hydrophobe Gruppe mit mindestens 8 Kohlenstoffatomen ist; und
· nichtionischen Monomeren vom Urethantyp, die bei der Reaktion von nichtionischen grenzflächenaktiven Stoffen mit einer Hydroxygruppe und monoethylenisch ungesättigten Isocyanaten gebildet werden;
- und gegebenenfalls einem Monomer zur Vernetzung;
wobei die prozentualen Gewichtsanteile der Monomere auf dem Gesamtgewicht der Monomere, die das Terpolymer bilden, basieren,
und dadurch, dass das Antischuppenmittel unter den folgenden Verbindungen ausgewählt ist:
1) Benzethoniumchlorid, Benzalkoniumchlorid, Chlorhexidin, Chloramin T, Chloramin B, 1,3-Dibrom-5,5-dimethylhydantoin, 1,3-Dichlor-5,5-dimethylhydantoin, 3-Brom-1-chlor-5,5-dimethylhydantoin, N-Chlorsuccinimid,
2) Derivaten von 1-Hydroxy-2-pyridon, die durch die folgende Formel (I) dargestellt werden können: 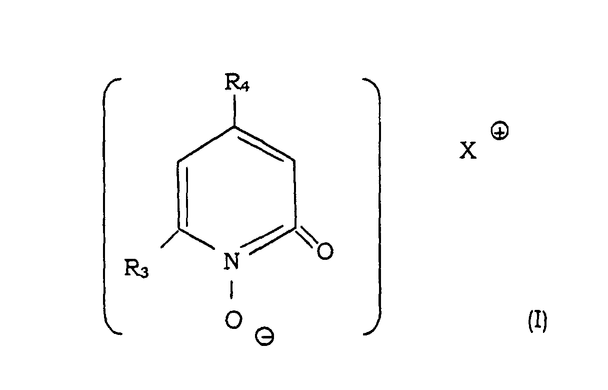 worin R₃ eine Alkylgruppe mit 1 bis 17 Kohlenstoffatomen, eine Alkenylgruppe mit 2 bis 17 Kohlenstoffatomen, eine Cycloalkylgruppe mit 5 bis 8 Kohlenstoffatomen, eine Bicycloalkylgruppe mit 7 bis 9 Kohlenstoffatomen; Cycloalkyl(-alkyl), Aryl, Aralkyl, wobei die Alkylgruppe 1 bis 4 Kohlenstoffatome aufweist, Arylalkenyl, wobei die Alkenylgruppe 2 bis 4 Kohlenstoffatome aufweist, Aryloxyalkyl oder Arylmercaptoalkyl, wobei die Alkylgruppe 1 bis 4 Kohlenstoffatome aufweist, Furylalkenyl mit einer Alkenyl- oder einer Furylgruppe, die 2 bis 4 Kohlenstoffatome aufweist, Alkoxy mit 1 bis 4 Kohlenstoffatomen, Nitro, Cyano oder ein Halogenatom bedeutet;
R₄ ein Wasserstoffatom, C₁₋₄-Alkyl, C₂₋₄-Alkenyl, ein Halogenatom, Phenyl oder Benzyl bedeutet; und
X eine organische Base, ein Alkalimetall- oder Erdalkalimetallion oder ein Ammoniumion bedeutet;
und diesen Derivaten in Form der Salze mit organischen oder anorganischen Basen;
3) Trihalogencarbamiden der folgenden Formel (II): 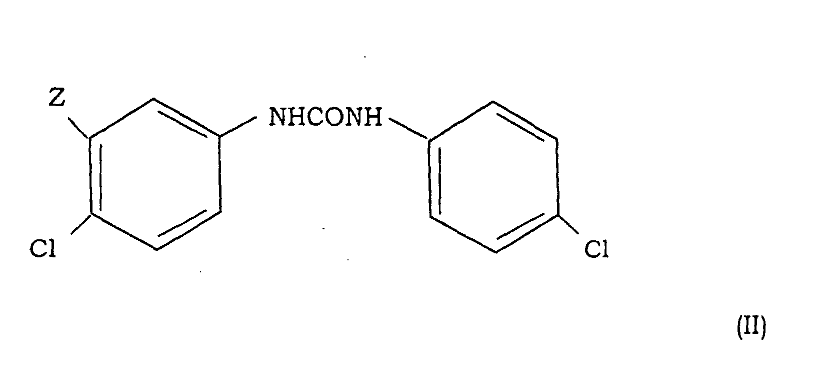 worin Z ein Halogenatom, wie Chlor oder eine Trihalogenalkylgruppe mit 1 bis 4 Kohlenstoffatomen bedeutet, wie CF₃;
4) Triclosan der Formel (III) 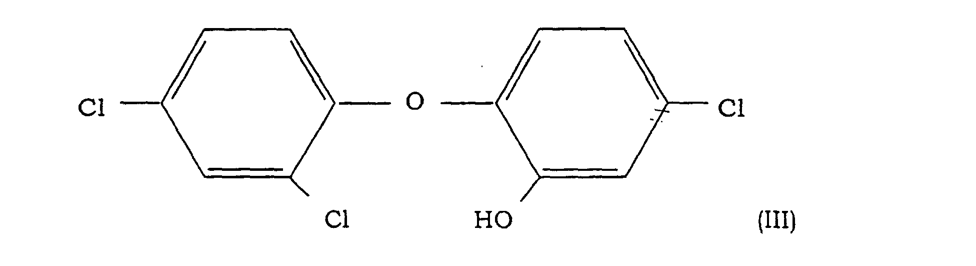
5) stickstoffhaltigen Verbindungen, wie Climbazol, Ketokonazol, Clotrinazol, Econazol, Isoconazol und Miconazol;
6) antimykotisch wirkenden Polymeren, wie Amphotericin B oder Nystatin;
7) Selensulfiden;
8) Schwefel in unterschiedlichen Formen, Cadmiumsulfid, Allantoin, Steinkohlenteer, Holzteer , Undecylensäure, Fumarsäure und Allylamine; oder
9) ein Gemisch dieser Antischuppenmittel.

2. Zusammensetzung nach Anspruch 1, **dadurch gekennzeichnet, dass** das Terpolymer in einer Menge von 0,01 bis 20 Gew.-% Wirkstoff und vorzugsweise 0,1 bis 10 Gew.-%, bezogen auf das Gesamtgewicht der Zusammensetzung, vorliegt.

3. Zusammensetzung nach Anspruch 1 oder 2, **dadurch gekennzeichnet, dass** das Monomer (a) unter den C₂₋₆-Alkylacrylaten ausgewählt ist und es sich vorzugsweise um Ethylacrylat handelt.

4. Zusammensetzung nach einem der Ansprüche 1 bis 3, **dadurch gekennzeichnet, dass** das Monomer (b) unter N,N-Dimethylaminoethylmethacrylat, N,N-Diethylaminoethylacrylat, N,N-Diethylaminoethylmethacrylat, N-t-Butylaminoethylacrylat, N-t-Butylaminoethylmethacrylat, N,N-Dimethylaminopropylacrylamid, N,N-Dimethylaminopropylmethacrylamid, N,N-Diethylaminopropylacrylamid und N,N-Diethylaminopropylmethacrylamid und vorzugsweise dem N,N-Dimethylaminoethylmethacrylat ausgewählt ist.

5. Zusammensetzung nach einem der Ansprüche 1 bis 4, **dadurch gekennzeichnet, dass** das Monomer (c) ein copolymerisierbares, ethylenisch ungesättigtes Tensidmonomer ist, das durch Kondensation eines nichtionischen grenzflächenaktiven Stoffes mit Itaconsäure entsteht.

6. Zusammensetzung nach einem der Ansprüche 1 bis 5, **dadurch gekennzeichnet, dass** das Acrylterpolymer aus Acrylaten, Amino(meth)acrylaten und mit 20 mol Ethylenoxid polyethoxyliertem C₁₀₋₃₀-Alkylitaconat besteht.

7. Zusammensetzung nach einem der Ansprüche 1 bis 6, **dadurch gekennzeichnet, dass** das Acrylterpolymer ferner ein Monomer zur Vernetzung enthält.

8. Zusammensetzung nach einem der Ansprüche 1 bis 7, **dadurch gekennzeichnet, dass** das Antischuppenmittel in einer Menge von 0,001 bis 10 Gew.-% und vorzugsweise 0,01 bis 5 Gew.-%, bezogen auf das Gesamtgewicht der Zusammensetzung, enthalten ist.

9. Zusammensetzung nach einem der Ansprüche 1 bis 8, **dadurch gekennzeichnet, dass** sie ferner mindestens einen grenzflächenaktiven Stoff enthält, der unter den anionischen, amphoteren, nichtionischen und kationischen grenzflächenaktiven Stoffen und deren Gemischen ausgewählt ist.

10. Zusammensetzung nach Anspruch 9, **dadurch gekennzeichnet, dass** die anionischen grenzflächenaktiven Stoffe unter den Alkalisalzen, Magnesiumsalzen, Ammoniumsalzen, Aminsalzen oder Aminoalkoholsalzen der folgenden Verbindungen: Alkylsulfate, Alkylethersulfate, Alkylamidoethersulfate, Alkylarylpolyethersulfate, Monoglyceridsulfate; Alkylsulfonate, Alkylamidsulfonate, Alkylarylsulfonate, Olefinsulfonate, Paraffinsulfonate; Alkylsulfosuccinate, Alkylethersulfosuccinate, Alkylamidsulfosuccinate; Alkylsulfosuccinamate; Alkylsulfoacetate; Alkylphosphate, Alkyletherphosphate; Acylsarcosinate, Acylisethionate und N-Acyltaurate; wobei die Alkyl- oder Acylgruppen dieser verschiedenen Verbindungen aus einer Kohlenstoffkette mit 8 bis 30 Kohlenstoffatomen bestehen; Salzen von Ölsäure, Ricinolsäure, Palmitinsäure und Stearinsäure; Säuren von Kopraöl oder hydriertem Kopraöl; Acyllactylaten, deren Acylgruppe 8 bis 30 Kohlenstoffatome aufweist; Alkyl-D-galactosiduronsäuren und ihren Salzen, polyalkoxylierten Carbonsäurealkylethern oder Carbonsäurealkylarylethern oder ihren Salzen und polyalkoxylierten Carbonsäurealkylamidoethem oder ihren Salzen ausgewählt sind.

11. Zusammensetzung nach Anspruch 9, **dadurch gekennzeichnet, dass** die nichtionischen grenzflächenaktiven Stoffe unter den Alkoholen oder Alkylphenolen oder Fettsäuren, die polyethoxyliert, polypropoxyliert oder mehrfach mit Glycerin verethert sind und eine Fettkette mit 8 bis 30 Kohlenstoffatomen aufweisen, wobei die Zahl der Ethylenoxid- und Propylenoxidgruppen im Bereich von 2 bis 50 und die Anzahl der Glyceringruppen im Bereich von 2 bis 30 liegt; den Copolymeren von Ethylenoxid und Propylenoxid; den Kondensaten von Ethylenoxid und Propylenoxid mit Fettalkoholen; polyethoxylierten Fettamiden; mehrfach mit Glycerin veretherten Fettamiden; polyethoxylierten Fettaminen; ethoxylierten Sorbitanfettsäureestern; Saccharosefettsäureestern oder Polyethylenglykolfettsäureestern; Alkylpolyglykosiden; A-mid- oder Carbamatderivaten von N-Alkylglucaminen, Aldobionamiden und Aminoxiden ausgewählt sind.

12. Zusammensetzung nach Anspruch 9, **dadurch gekennzeichnet, dass** die amphoteren grenzflächenaktiven Stoffe unter den aliphatischen, sekundären oder tertiären Aminderivaten, deren aliphatische Gruppe eine lineare oder verzweigte Kette mit 8 bis 22 Kohlenstoffatomen ist, die mindestens eine wasserlösliche anionische Gruppe, Carboxylat, Sulfonat, Sulfat, Phosphat oder Phosphonat aufweist; C₈₋₂₀-Alkylbetainen; Sulfobetainen, C₈₋₂₀-Alkyl-C₁₋₆-amidoalkylbetainen oder C₈₋₂₀-Alkyl-C₁₋₆-amidoalkylsulfobetainen ausgewählt sind.

13. Zusammensetzung nach Anspruch 9, **dadurch gekennzeichnet, dass** die kationischen grenzflächenaktiven Stoffe unter den primären, sekundären oder tertiären, gegebenenfalls polyalkoxylierten Aminsalzen; quartären Ammoniumsalzen; Imidazolinderivaten; und Aminoxiden mit kationischem Charakter ausgewählt sind.

14. Zusammensetzung nach Anspruch 9, **dadurch gekennzeichnet, dass** der grenzflächenaktive Stoff in einer Menge von 0,01 bis 50 Gew.-%, bezogen auf das Gesamtgewicht der Zusammensetzung, enthalten ist.

15. Zusammensetzung nach Anspruch 14, **dadurch gekennzeichnet, dass** der grenzflächenaktive Stoff in einer Menge von mindestens 4 Gew.-%, vorzugsweise 5 bis 50 Gew.-% und noch bevorzugter 8 bis 35 Gew.-%, bezogen auf das Gesamtgewicht der Zusammensetzung, enthalten ist.

16. Zusammensetzung nach einem der Ansprüche 1 bis 15, **dadurch gekennzeichnet, dass** sie einen pH-Wert im Bereich von 3 bis 12 und insbesondere 4 bis 8 aufweist.

17. Zusammensetzung nach einem der Ansprüche 1 bis 16, **dadurch gekennzeichnet, dass** das kosmetisch akzeptable Medium aus Wasser oder einem oder mehreren Lösungsmitteln oder einem Gemisch von Wasser und mindestens einem kosmetisch akzeptablen Lösungsmittel besteht, das unter den niederen Alkoholen, Alkylenglykolen und Polyolethern ausgewählt ist.

18. Zusammensetzung nach einem der Ansprüche 1 bis 17, **dadurch gekennzeichnet, dass** sie ferner mindestens ein Polyorganosiloxan enthält, das unter den flüchtigen Siliconen und nicht flüchtigen Siliconen und insbesondere den folgenden Verbindungen ausgewählt ist:
(i) Polyalkylsiloxanen;
(ii) Polyarylsiloxanen;
(iii) Polyalkylarylsiloxanen;
(iv) Silicongummis;
(v) Siliconharzen;
(vi) organomodifizierten Polyorganosiloxanen;
(vii) Blockcopolymeren, die als wiederkehrende Einheit einen linearen Polysiloxan-Polyalkylen-Block enthalten;
(viii) gepfropften Siliconpolymeren mit organischem, nicht siliconhaltigem Grundgerüst;
(ix) gepfropften Siliconpolymeren mit Polysiloxangrundgerüst, auf das nicht siliconhaltige, organische Monomere gepfropft sind;
(x) und deren Gemischen.

19. Zusammensetzung nach Anspruch 18, **dadurch gekennzeichnet, dass** sie ein Polyorganosiloxan in Mengenanteilen von 0,01 bis 20 Gew.-% und vorzugsweise 0,1 bis 10 Gew.-%, bezogen auf das Gesamtgewicht der Zusammensetzung, enthält.

20. Zusammensetzung nach einem der Ansprüche 1 bis 19, **dadurch gekennzeichnet, dass** sie ferner mindestens ein kationisches Polymer enthält, das ausgewählt ist unter:
. Proteinen oder Proteinhydrolysaten, insbesondere Kollagenhydrolysaten mit Triethylammoniumgruppen, Kollagenhydrolysaten mit Trimethylammoniumchlorid- und Trimethylstearylammoniumchloridgruppen, Proteinhydrolysaten, die an der Polypeptidkette quartäre Ammoniumgruppen tragen, die mindestens eine C₁₋₁₈-Alkylgruppe enthalten, quaternisierten pflanzlichen Proteinen, wie Weizenproteinen, Maisproteinen oder Sojaproteinen,
. Polymeren vom Polyamintyp, Polyaminoamidtyp oder quartären Polyammoniumtyp, insbesondere
(i) Copolymeren von Vinylpyrrolidon und Dialkylaminoalkylacrylat oder Dialkylaminoalkylmethacrylat, die gegebenenfalls quatemisiert sind,
(ii) Derivaten von Celluloseethern, die quartäre Ammoniumgruppen enthalten,
(üi) kationischen Cellulosederivaten, wie Cellulosecopolymeren oder Cellulosederivaten, die mit einem wasserlöslichen quartären Ammoniumpolymer gepfropft sind,
(iv) Polysacchariden und insbesondere kationischen Guargummen,
(v) Polymeren, die aus Piperazinyleinheiten und zweiwertigen Alkylen- oder Hydroxyalkylengruppen mit geraden oder verzweigten Ketten bestehen, die gegebenenfalls durch Sauerstoffatome, Schwefelatome, Stickstoffatome oder aromatische oder heterocyclische Ringe unterbrochen sind, sowie den Oxidationsprodukten und/oder Quaternisierungsprodukten dieser Polymere,
(vi) wasserlöslichen Polyaminoamiden, die insbesondere durch Polykondensation einer sauren Verbindung mit einem Polyamin hergestellt werden,
(vii) Derivaten von Polyaminoamiden, die bei der Kondensation von Polyalkylenpolyaminen mit Polycarbonsäuren und anschließender Alkylierung mit bifunktionellen Mitteln entstehen,
(viii) Polymeren, die durch Umsetzung eines Polyalkylenpolyamins, das zwei primäre Aminogruppen und mindestens eine sekundäre Aminogruppe aufweist, mit einer Dicarbonsäure hergestellt sind, welche unter Diglykolsäure und den gesättigten, aliphatischen Dicarbonsäuren mit 3 bis 8 Kohlenstoffatomen ausgewählt ist,
(ix) Methyldiallylamin- oder Dimethyldiallylammonium-Cyclopolymeren,
(x) quartären Diammoniumpolymeren,
(xi) quartären Polyammoniumpolymeren,
(xii) Homo- oder Copolymeren, die von Acrylsäure oder Methacrylsäure abgeleitet sind und die Einheiten CH₂-CHRₐ-CO-O-A₁-NRₑR_{f}, CH₂-CHRₐ-CO-O-A₁-N⁺R_{b}R_{c}R_{d}X- und/oder CH₂-CHRₐ-CO-NH-A₁-N⁺R_{b}R_{c}R_{d}X⁻ enthalten, wobei die Gruppen Rₐ unabhängig voneinander H oder CH₃ bedeuten, die Gruppen A₁ unabhängig voneinander eine geradkettige oder verzweigte Alkylgruppe mit 1 bis 6 Kohlenstoffatomen oder eine Hydroxyalkylgruppe mit 1 bis 4 Kohlenstoffatomen bedeuten, die Gruppen R_{b}, R_{c} und R_{d}, die identisch oder voneinander verschieden sind, unabhängig voneinander eine Alkylgruppe mit 1 bis 18 Kohlenstoffatomen oder Benzyl bedeuten, die Gruppen Rₑ und R_{f} Wasserstoff oder eine Alkylgruppe mit 1 bis 6 Kohlenstoffatomen bedeuten und X- ein Anion ist, beispielsweise Methosulfat oder ein Halogenid, wie Chlorid oder Bromid,
(xiii) quartären Polymeren von Vinylpyrrolidon und Vinylimidazol,
(xiv) Polyaminen, die nach CTFA-Nomenklatur als "POLYETHYLENEGLYCOL TALLOW POLYAMINE" bezeichnet werden, und
(xv) vernetzten Polymeren von Methacryloyloxyethyltrimethylammoniumchlorid,
. Polyalkyleniminen, Polymeren, die Vinylpyridin- oder Vinylpyridinium-Einheiten enthalten, Kondensaten von Polyaminen und Epichlorhydrin, quartären Polyureylenen und Chitinderivaten.

21. Zusammensetzung nach Anspruch 20, **dadurch gekennzeichnet, dass** sie ein kationisches Polymer in Mengenanteilen von 0,001 bis 20 Gew.-% und vorzugsweise 0,05 bis 5 Gew.-%, bezogen auf das Gesamtgewicht der Zusammensetzung, enthält.

22. Zusammensetzung nach einem der Ansprüche 1 bis 20, **dadurch gekennzeichnet, dass** sie außerdem mindestens einen kosmetisch akzeptablen Zusatzstoff enthält, der unter Parfums, Konservierungsmitteln, Maskierungsmitteln, Feuchthaltemitteln, Zuckern, pflanzlichen, mineralischen, tierischen oder synthetischen Ölen, amphoteren Polymeren, Menthol, Nicotinatderivaten, Wirkstoffen gegen Haarausfall, Schaumstabilisatoren, Treibmitteln, Farbmitteln, Filtern, Ceramiden, Vitaminen oder Provitaminen und Ansäuerungs- oder Alkalisierungsmitteln ausgewählt ist.

23. Kosmetische Verwendung einer Zusammensetzung nach einem der Ansprüche 1 bis 22 als Haarwaschmittel, Lotion oder Produkt, das nach der Haarwäsche aufgetragen wird.

24. Kosmetisches Verfahren zur Behandlung der Haare, **dadurch gekennzeichnet, dass** auf die feuchten oder trockenen Haare mindestens eine Zusammensetzung nach einem der Ansprüche 1 bis 22 aufgetragen und nach einer fakultativen Einwirkzeit gegebenenfalls mit Wasser gespült wird.

## Claims

1. Antidandruff composition for treating the hair and the scalp, comprising at least one antidandruff agent in a cosmetically acceptable medium, **characterized in that** it also comprises at least one acrylic terpolymer consisting of:
- from 5% to 80% by weight, preferably from 15% to 70% by weight and more preferably from 40% to 70% by weight of an acrylate monomer (a) chosen from a C₁-C₆ alkyl acrylate and a C₁-C₆ alkyl methacrylate;
- from 5% to 80% by weight, preferably from 10% to 70% by weight and more preferably from 20% to 60% by weight, of a monomer (b) chosen from a heterocyclic vinyl compound containing at least one nitrogen or sulphur atom, a (meth)acrylamide, a mono- or di (C₁-C₄)alkylamino (C₁-C₄)alkyl (meth)acrylate and a mono- or di (C₁-C₄) alkylamino (C₁-C₄) alkyl (meth) acrylamide;
- from 0.1% to 30% by weight, preferably from 0.1% to 10% by weight, of a monomer (c) chosen from:
a urethane produced by reaction between a monoethylenic unsaturated isocyanate and a nonionic surfactant comprising a block copolymer of 1,2-butylene oxide and of ethylene oxide with a C₁₋₆ alkoxy end;
a copolymerizable ethylenic unsaturated surfactant monomer obtained by condensation of a nonionic surfactant with an α,β-ethylenic unsaturated carboxylic acid or its anhydride;
a surfactant monomer chosen from the products of reaction of the type such as a urea of a monoethylenic unsaturated monoisocyanate with a nonionic surfactant containing an amine function;
a (meth)allyl ether of formula CH₂=CR₁CH₂OAₘBₙAₚR₂ in which R₁ denotes a hydrogen atom or a methyl group, A denotes a propylenoxy or butylenoxy group, B denotes ethylenoxy, n is equal to zero or denotes an integer less than or equal to 200, m and p denote zero or an integer less than n and R₂ is a hydrophobic group of at least 8 carbon atoms; and
a nonionic monomer of urethane type produced by reaction of a monohydric nonionic surfactant with a monoethylenic unsaturated isocyanate;
and optionally a crosslinking monomer
the weight percentages of monomers being based on the total weight of the monomers constituting the terpolymer,
and **in that** the antidandruff agent is chosen from:
1) benzethonium chloride, benzalkonium chloride, chlorhexidine, chloramine T, chloramine B, 1,3-dibromo-5,5-dimethylhydantoin, 1,3-dichloro-5,5-dimethylhydantoin, 3-bromo-1-chloro-5,5-dimethylhydantoin and N-chlorosuccinimide;
2) the 1-hydroxy-2-pyridone derivatives represented by formula (I): 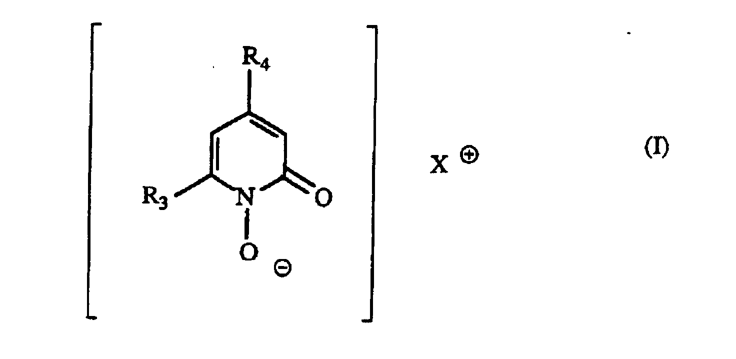 in which R₃ represents an alkyl group containing from 1 to 17 carbon atoms, an alkenyl group containing from 2 to 17 carbon atoms, a cycloalkyl group containing from 5 to 8 carbon atoms or a bicycloalkyl group containing from 7 to 9 carbon atoms; a cycloalkyl(-alkyl) group, an aryl group, an aralkyl group with an alkyl containing from 1 to 4 carbon atoms, an arylalkenyl group with an alkenyl containing from 2 to 4 carbon atoms, an aryloxyalkyl or arylmercaptoalkyl group with an alkyl containing from 1 to 4 carbon atoms, a furylalkenyl group with an alkenyl or a furyl containing from 2 to 4 carbon atoms, an alkoxy group containing from 1 to 4 carbon atoms, a nitro group, a cyano group or a halogen atom;
R₄ represents a hydrogen atom, a C₁-C₄ alkyl group, a C₂-C₄ alkenyl group, a halogen atom, a phenyl group or a benzyl group; X represents an organic base, an alkali metal ion, an alkaline-earth metal ion or an ammonium ion;
and these derivatives are in the form of salts with organic or inorganic bases;
3) the trihalocarbamides of formula (II) below: 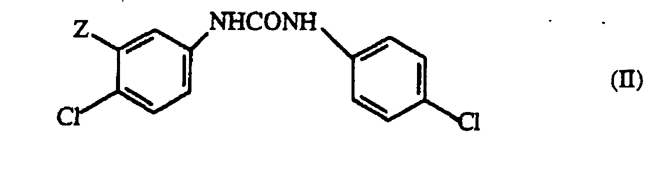 in which Z represents a halogen atom such as chlorine or a C₁-C₄ trihaloalkyl group such as CF₃;
4) triclosan, represented by formula (III) 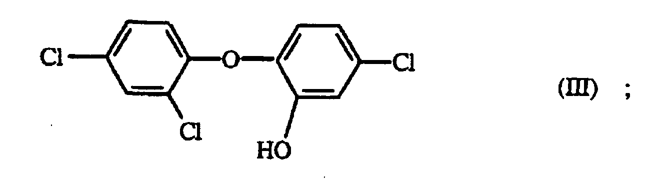
5) azole compounds such as climbazole, ketoconazole, clotrinazole, econazole, isoconazole and miconazole;
6) antifungal polymers such as amphotericin B or nystatin;
7) selenium sulphides;
8) sulphur in its various forms, cadmium sulphide, allantoin, coal tar or wood tar, undecylenic acid, fumaric acid, and allylamines;
9) a mixture of these antidandruff agents.

2. Composition according to Claim 1, **characterized in that** the terpolymer is present in a proportion of from 0.01% to 20% by weight of active material, preferably 0.1% to 10% by weight, relative to the total weight of the composition.

3. Composition according to Claim 1 or 2, **characterized in that** the monomer (a) is chosen from C₂-C₆ alkyl acrylates and is preferably ethyl acrylate.

4. Composition according to any one of Claims 1 to 3, **characterized in that** the monomer (b) is chosen from N,N-dimethylaminoethyl methacrylate, N,N-diethylaminoethyl acrylate, N,N-diethylaminoethyl methacrylate, N-t-butylaminoethyl acrylate, N-t-butylaminoethyl methacrylate, N,N-dimethylaminopropylacrylamide, N,N-dimethylaminopropylmethacrylamide, N,N-diethylaminopropylacrylamide and N,N-diethylaminopropylmethacrylamide and is preferably N,N-dimethylaminoethyl methacrylate.

5. Composition according to any one of Claims 1 to 4, **characterized in that** the monomer (c) is a copolymerizable ethylenic unsaturated surfactant monomer obtained by condensing a nonionic surfactant with itaconic acid.

6. Composition according to any one of Claims 1 to 5, **characterized in that** the acrylic terpolymer consists of acrylates, amino (meth)acrylates and C₁₀-C₃₀ alkyl itaconate, polyoxyethylenated with 20 mol of ethylene oxide.

7. Composition according to any one of Claims 1 to 6, **characterized in that** the acrylic terpolymer also contains a crosslinking monomer.

8. Composition according to any one of Claims 1 to 7, **characterized in that** the antidandruff agent is present in a proportion of from 0.001% to 10% by weight, preferably from 0.01% to 5% by weight, relative to the total weight of the composition.

9. Composition according to any one of Claims 1 to 8, **characterized in that** it also contains at least one surfactant chosen from anionic, amphoteric, nonionic and cationic surfactants and mixtures thereof.

10. Composition according to Claim 9, **characterized in that** the anionic surfactants are chosen from alkaline salts, magnesium salts, ammonium salts, amine salts or amino alcohol salts of the following compounds: alkyl sulphates, alkyl ether sulphates, alkylamido ether sulphates, alkylaryl polyether sulphates, monoglyceride sulphates; alkyl sulphonates, alkylamide sulphonates, alkylaryl sulphonates, olefin sulphonates, paraffin sulphonates; alkyl sulphosuccinates, alkyl ether sulphosuccinates, alkylamide sulphosuccinates; alkyl sulphosuccinamates; alkyl sulphoacetates; alkyl phosphates, alkyl ether phosphates; acyl sarcosinates, acyl isethionates and N-acyl taurates; the alkyl or acyl radical in these various compounds generally consisting of a carbon-based chain containing from 8 to 30 carbon atoms; the fatty acid salts of oleic, ricinoleic, palmitic and stearic acid salts; coconut oil acid or hydrogenated coconut oil acid; acyl lactylates, in which the acyl radical contains from 8 to 30 carbon atoms; alkyl D-galactosiduronic acids and salts thereof, polyoxyalkylenated alkyl or alkylaryl ether carboxylic acids or salts thereof, and polyoxyalkylenated alkylamido ether carboxylic acids or salts thereof.

11. Composition according to Claim 9, **characterized in that** the nonionic surfactants are chosen from polyethoxylated, polypropoxylated or polyglycerolated fatty acids or alkylphenols or alcohols, with a fatty chain containing 8 to 30 carbon atoms, the number of ethylene oxide or propylene oxide groups being between 2 and 50 and the number of glycerol groups being between 2 and 30; the copolymers of ethylene oxide and propylene oxide; condensates of ethylene oxide and of propylene oxide with fatty alcohols; polyethoxylated fatty amides; polyglycerolated fatty amides; polyethoxylated fatty amines; oxyethylenated fatty acid esters of sorbitan; fatty acid esters of sucrose or of polyethylene glycol; alkylpolyglycosides; carbamate or amide derivatives of N-alkylglucamines, aldobionamides, and amine oxides.

12. Composition according to Claim 9, **characterized in that** the amphoteric surfactants are chosen from secondary or tertiary aliphatic amine derivatives, in which the aliphatic radical is a linear or branched chain containing 8 to 22 carbon atoms and which contains at least one carboxylate, sulphonate, sulphate, phosphate or phosphonate water-solubilizing anionic group; (C₈-C₂₀)alkylbetaines, sulphobetaines, (C₈-C₂₀)alkylamido(C₁-C₆)alkylbetaines or (C₈-C₂₀)alkylamido (C₁-C₆)alkylsulphobetaines.

13. Composition according to Claim 9, **characterized in that** the cationic surfactants are chosen from optionally polyoxyalkylenated primary, secondary or tertiary fatty amine salts; quaternary ammonium salts; imidazoline derivatives; and amine oxides of cationic nature.

14. Composition according to Claim 9, **characterized in that** the surfactant is present in a proportion of from 0.01% to 50% by weight relative to the total weight of the composition.

15. Composition according to Claim 14, **characterized in that** the surfactant is present in a proportion of at least 4% by weight, preferably from 5% to 50% by weight and even more preferably from 8% to 35% by weight, relative to the total weight of the composition.

16. Composition according to any one of Claims 1 to 15, **characterized in that** it has a pH of between 3 and 12 and more particularly between 4 and 8.

17. Composition according to any one of Claims 1 to 16, **characterized in that** the cosmetically acceptable medium consists of water or one or more solvents or of a mixture of water and at least one cosmetically acceptable solvent chosen from lower alcohols, alkylene glycols and polyol ethers.

18. Composition according to any one of Claims 1 to 17, **characterized in that** it also contains at least one polyorganosiloxane chosen from volatile silicones and non-volatile silicones and in particular
(i) polyalkylsiloxanes;
(ii) polyarylsiloxanes;
(iii) polyalkylarylsiloxanes;
(iv) silicone gums;
(v) silicone resins;
(vi) organomodified polyorganosiloxanes;
(vii) block copolymers containing a linear polysiloxane-polyalkylene block as repeating unit;
(viii) grafted silicone polymers, containing a non-silicone organic skeleton;
(ix) grafted silicone polymers, containing a polysiloxane skeleton grafted with non-silicone organic monomers;
(x) and mixtures thereof.

19. Composition according to Claim 18, **characterized in that** it contains a polyorganosiloxane in proportions of between 0.01% and 20% by weight and preferably between 0.1% and 10% by weight relative to the total weight of the composition.

20. Composition according to any one of Claims 1 to 19, **characterized in that** it also contains at least one cationic polymer chosen from:
- proteins or protein hydrolysates, in particular collagen hydrolysates bearing triethylammonium groups, collagen hydrolysates bearing trimethylammonium chloride and trimethylstearylammonium chloride groups, protein hydrolysates bearing on the polypeptide chain quaternary ammonium groups comprising at least one alkyl radical containing from 1 to 18 carbon atoms, and quaternized plant proteins such as wheat, corn or soybean proteins;
- polymers of the polyamine, polyaminoamide or polyquaternary ammonium type, in particular
i) Quaternized or non-quaternized vinyl-pyrrolidone/dialkylaminoalkyl acrylate or methacrylate copolymers,
ii) cellulose ether derivatives comprising quaternary ammonium groups,
iii) cationic cellulose derivatives such as cellulose copolymers or cellulose derivatives grafted with a water-soluble quaternary ammonium monomer,
iv) polysaccharides and in particular cationic guar gums,
v) polymers consisting of piperazinyl units and of divalent alkylene or hydroxyalkylene radicals containing straight or branched chains, optionally interrupted with oxygen, sulphur or nitrogen atoms or with aromatic or heterocyclic rings, as well as the oxidation and/or quaternization products of these polymers,
vi) water-soluble polyaminoamides prepared in particular by polycondensation of an acidic compound with a polyamine,
vii) polyaminoamide derivatives resulting from the condensation of polyalkylene polyamines with polycarboxylic acids followed by an alkylation with bifunctional agents,
viii) polymers obtained by reacting a polyalkylene polyamine comprising two primary amine groups and at least one secondary amine group with a dicarboxylic acid chosen from diglycolic acid and saturated aliphatic dicarboxylic acids containing from 3 to 8 carbon atoms,
ix) cyclopolymers of methyldiallylamine or of dimethyldiallylammonium,
x) the diquaternary ammonium polymers,
xi) the polyquaternary ammonium polymers,
xii) homopolymers or copolymers derived from acrylic or methacrylic acids and comprising CH₂-CHRₐ-CO-O-A₁-NRₑR_{f}, CH₂-CHRₐ-CO-O-A₁-N⁺R_{b}R_{c}R_{d}, X⁻ and/or CH₂-CHRₐ-CO-NH-A₁-N⁺R_{b}R_{c}R_{d}, X⁻ units,
in which the groups Rₐ independently denote H or CH₃, the groups A₁ independently denote a linear or branched alkyl group of 1 to 6 carbon atoms or a hydroxyalkyl group of 1 to 4 carbon atoms, the groups R_{b}, R_{c} and R_{d}, which may be identical or different, independently denote an alkyl group of 1 to 18 carbon atoms or a benzyl radical, the groups Rₑ and R_{f} represent a hydrogen atom or an alkyl group of 1 to 6 carbon atoms, X⁻ denotes an anion, for example methosulphate or halide, such as chloride or bromide,
xiii) quaternary polymers of vinylpyrrolidone and of vinylimidazole,
xiv) the polyamines referred to under the name "Polyethylene Glycol Tallow Polyamine" in the CTFA dictionary,
xv) crosslinked methacryloyloxyethyltrimethyl-ammonium chloride polymers,
- polyalkyleneimines, polymers containing vinylpyridine or vinylpyridinium units, condensates of polyamines and of epichlorohydrin, polyquaternary ureylenes and chitin derivatives.

21. Composition according to Claim 20, **characterized in that** it contains a cationic polymer in proportions of between 0.001% and 20% by weight and preferably between 0.05% and 5% by weight relative to the total weight of the composition.

22. Composition according to any one of Claims 1 to 20, **characterized in that** it also contains at least one cosmetically acceptable adjuvant chosen from fragrances, preserving agents, screening agents, sequestering agents, wetting agents, sugars, plant, animal, mineral or synthetic oils, amphoteric polymers, menthol, nicotinate derivatives, agents for preventing hair loss, foam stabilizers, propellants, dyes, ceramides, vitamins or provitamins and acidifying or basifying agents.

23. Cosmetic use, as a shampoo, lotion or conditioner, of a composition as defined in any one of Claims 1 to 22.

24. Cosmetic hair treatment process, **characterized in that** at least one composition as defined in any one of Claims 1 to 22 is applied to wet or dry hair and, after optionally leaving the composition on the hair for a period of time, the hair is optionally rinsed with water.
